# EUROPEAN PATENT APPLICATION

(11) **EP 4 806 448 A1**
(43) Date of publication of application: **16.09.2026**
(21) Application number: 23958392.5
(22) Date of filing: 09.11.2023
(51) Int. Cl.: A61K 36/48, A61K 36/8962, A61K 36/31, A61K 35/20, A61K 38/06, A61K 31/192, A61P 9/12, A61P 1/00, A23L 33/105

(54) **COMPOSITION FOR INHIBITING PRODUCTION OF CARCINOGENIC N-NITROSAMINE IN STOMACH AND USE THEREOF**

(71) Applicant: Chun, Hyun-Soo, Seoul 02573 (KR); Humanenos LLC, Jeonbuk-do 55347 (KR)
(72) Inventor: CHUN, Hyun-Soo, Seoul 02573 (KR)
(74) Representative: Gulde & Partner
(86) International application number: PCT/KR2023/017930
(87) International publication number: WO 2025/100593

(57) **Abstract**

The present invention relates to a composition for preventing, ameliorating, or treating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract. The composition of the present invention can generate S-nitrosoglutathione under gastric juice conditions (strongly acidic) in the stomach, be delivered to the small or large intestine, and produce nitric oxide at a certain concentration for a specific period of time, making it suitable for various applications.

## Description

### TECHNICAL FIELD

The present invention relates to a composition for inhibiting the formation of carcinogenic N-nitrosamines in the stomach and a use thereof, and more specifically, to a composition capable of blocking the formation of carcinogenic N-nitrosamines under strongly acidic conditions such as in the stomach, and generating a certain amount of nitric oxide in the intestinal environment to enable absorption of nitric oxide metabolites in the body.

### BACKGROUND ART

Nitric oxide (nitric oxide, hereinafter referred to as NO) is a gaseous substance produced by nitric oxide synthase (NOS), which is present in bacteria, plants, and animals, through decomposition of the amino acid known as arginine (L-arginine), and is a highly reactive radical (see Reaction 1-1)). NO is a hydrophobic and unstable substance having chemical properties such that it is rapidly oxidized and converted into hydrophilic NO metabolites (referring to nitrite ion [NO₂⁻], nitrate ion [NO₃⁻], and the like) (see Reactions 1-2) and 1-3)). NO has a very small molecular size and is hydrophobic, and thus is capable of rapidly diffusing in aqueous environments such as blood or bodily fluids. In particular, NO can pass through vascular barriers and cell membranes within its given lifetime (estimated to be less than about 1 second). In addition, since the generated NO is highly reactive, it reacts with oxygen dissolved in blood or bodily fluids or with oxygen bound to red blood cells, and is thereby converted (oxidized) into water-soluble NO metabolites having a longer lifetime (see Reactions 1-2) and 1-3)). As a result, such metabolites are dissolved in aqueous blood, can be transported throughout the body, and can be excreted from the body.

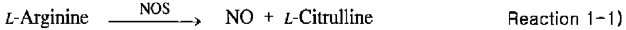

**4NO** + O₂ + 2H₂O → 4NO₂⁻ + 4H⁺ Reaction 1-2

)

NO + Hb(II)O₂ → NO₃⁻ + Hb(III) Reaction 1-3)

^{Footnote)}Hb(II)O₂; oxy-hemoglobin, Hb(III); deoxy-hemoglobin

As an NO metabolite, NO₂⁻ is a hydrophilic and relatively stable ionic substance, and although it is dissolved in blood and bodily fluids and can be transported throughout the body, it cannot pass through organ barriers, vascular barriers, or cell membranes. However, NO₂⁻ is reduced to hydrophobic NO under acidic conditions and hypoxic conditions (e.g., venous blood conditions), thereby enabling it to pass through organ barriers, vascular barriers, and cell membranes and to exhibit the intrinsic physiological functions and pharmacological effects of NO (see Reaction 2-1)). In addition, NO₂⁻ may be reduced to NO by reacting with vitamin C (ascorbic acid/ascorbate, hereinafter referred to as Asc) dissolved in blood or bodily fluids (see Reactions 2-2) and 2-3)).

NO₂⁻ + Hb(II) + H⁺ → NO + Hb(III) + OR⁻ Reaction 2-1)

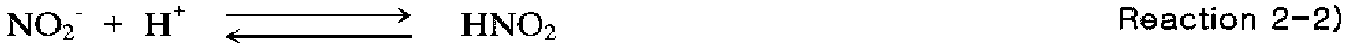

2HNO₂ + Asc → 2NO + dehydroAsc + H₂O Reaction 2-3)
^{Footnote1)} Hb(II); deoxy-hemoglobin, Ase; ascorbate, dehydroAsc; deoxyhyro-ascorbate
^{Footnota2)} H⁺ in Reactions 2-1) and 2-2) is provided from carbonic acid generated by dissolution of CO₂
^{Footnote3)} Vitamin C exists as ascorbate in blood or body fluids

In the human body, NO is mainly produced in vascular endothelial cells through the process of Reaction 1-1), and is used for regulating physiological functions such as relaxation of adjacent blood vessels. A portion of the generated NO is oxidized to NO₂⁻ through the process of Reaction 1-2) and dissolved in the blood, thereby being transported to all parts of the body, and is reduced to NO at sites in which NO is required through the process of Reaction 2-1) or Reaction 2-2), thereby exhibiting the physiological functions and pharmacological effects of NO. NO produced in excess by NOS or NO generated by reduction of excess NO₂⁻ is oxidized to NO₃⁻ through the process of Reaction 1-3) and is excreted from the body through urine or sweat. Therefore, it may be considered that there is no significant toxicity to the human body due to excess NO or NO₂⁻. However, in the process of Reaction 1-3), cyanosis may occur as red blood cells lose oxygen, but such symptoms are rapidly resolved as the red blood cells recombine with oxygen.

NO generated in Reaction 1-1) or 2-1) reaches the cytoplasm of target cells and chemically binds with the abundantly present glutathione (hereinafter referred to as GSH) in the cytoplasm to form S-nitrosoglutathione (hereinafter referred to as GSNO), thereby storing NO inside the cell in the form of GSNO for a certain period of time (see Reaction 3-1)). When the cell requires NO, the stored GSNO combines with GSH to generate NO and glutathione disulfide (hereinafter referred to as GSSG) (see Reaction 3-2)). Meanwhile, when GSNO is present in excess inside the cell, the excess GSNO is reduced by GSNO reductase (hereinafter referred to as GSNOR) to GSH and NH₃, thereby reducing the potential toxicity of NO. 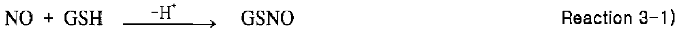 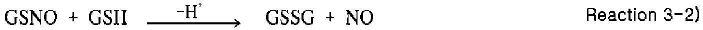 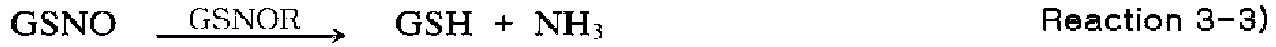
^{Footnote1)} GSH; glutathione, GSNO; S-nitrosoglutathione, GSNOR; GSNO reductase
^{Footnote2)} Reactions 3-1) and 3-2) occur spontaneously inside and outside the cells depending on their respective concentrations
^{Footnote3)} Reaction 3-3) occurs spontaneously inside the cells depending on the concentration of GSNO

The total amount of NO in the human body can be predicted by the combined amounts of NO₂⁻ and NO₃⁻, but the actual usable NO concentration can be estimated based on the concentration of NO₂⁻ that can be converted to NO in the blood. In the blood of healthy individuals without disease, NO₂⁻ is known to exist at approximately 100-500 nM (average 300 nM); however, in individuals with diseases such as diabetes, blood NO₂⁻ concentrations are often observed to be lower than those of healthy individuals. There may be various methods to restore reduced NO₂⁻ concentrations to the normal range, but the most reasonable approach is thought to be maintaining blood NO₂⁻ concentrations within the normal range by consuming NO metabolites through food or dietary intake. Indeed, humans and animals maintain their blood NO₂⁻concentrations within the normal range through dietary intake. Furthermore, analysis of multiple reports on the effects of NO metabolites in experimental animals shows that restricting NO metabolite intake induces diabetic symptoms in animals, whereas oral administration of NO metabolites to diabetic animals improved the condition (Kina-Tanada et al., Diabetologia. 2017, 60(6): 1138-1151).

Humans and experimental animals cannot directly absorb NO metabolites into the body. When humans ingest NO metabolites, the NO₂⁻ of the NO metabolites is reduced to NO under the strongly acidic conditions of the stomach. The reduced NO then passes through the stomach wall, reaches the blood or bodily fluids, and is oxidized back to NO₂⁻, thereby completing absorption in the body (see Reaction 4)).

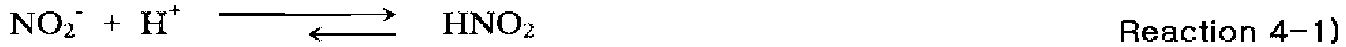

2HNO₂ → N₂O₃ Reaction 4-2)

N₂O₃ → NO + NO₂ Reaction 4-3)

^{Footnote1)}In Reaction 4-1), the reverse reaction does not proceed well because the conditions are strongly acidic
^{Footnote2)}In Reaction 4-2), the forward reaction proceeds well because N₂O₃ is hydrophobic
^{Footnote3)}In Reaction 4-3), N₂O₃ is an unstable reaction intermediate and spontaneously decomposes to produce NO

Meanwhile, ingested NO₃⁻ (another NO metabolite), unlike NO₂⁻, is first reduced to NO₂⁻ by NO₃⁻nitrate reductase (hereinafter referred to as NaRase) of symbiotic bacteria in the small and large intestines. The NO₂⁻ is then reduced to NO by NO₂⁻ nitrite reductase (hereinafter referred to as NiRase) present in the periplasm between the outer membrane and cytoplasmic membrane of the bacteria. A portion of the reduced NO is absorbed into the body, while the remaining NO undergoes several reduction steps and is ultimately converted to N₂ and eliminated in the intestines (see Reaction 5)). Therefore, in order to generate a sufficient amount of NO using NO₃⁻, a large intake of NO₃⁻ is required. 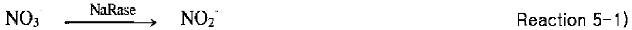 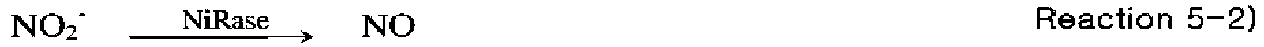 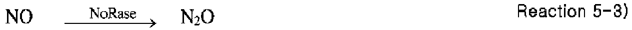 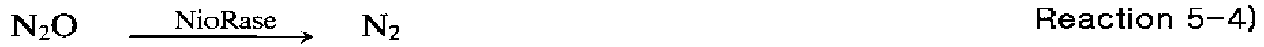
^{Footnote1)} NaRase, nitrate reductase; NiRase, nitrite reductase; NoRase, nitric oxide reductase; NioRase, nitrous oxide reductase
^{Footnote2)} A portion of the NO produced In Reaction 5-2) Is absorbed Into the body
^{Footnote3)} A portion of the NO is not absorbed into the body due to Reactions 5-3) and 5-4)
^{Footnote4)} Reactions 5-1) through 5-4) are known as denitrification reactions

When NO metabolites are orally administered to increase low blood NO (or NO₂⁻) concentrations, the following three health-hazardous problems may occur.

First, orally administered NO₂⁻ can rapidly cause absorption of an excessive amount of NO due to the strongly acidic conditions of gastric juice, which may lead to sudden blood pressure drops, hypoglycemia, cyanosis, or the like.

Second, orally administered NO₂⁻ can react with secondary amines (hereinafter referred to as R₂N-H) under the strongly acidic conditions of gastric juice to form N-nitrosamines (hereinafter referred to as R₂N-NO), which are known carcinogens (see Reaction 6)).

Third, orally administered NO₃⁻ is not reduced in the stomach but is reduced to NO in the small and large intestines and absorbed into the body; however, the extent of NO reduction varies depending on an individual's intestinal bacterial environment and dietary habits, and a greater amount of NO₃⁻ must be ingested compared to NO₂⁻. Therefore, oral intake of NO₃⁻ cannot be expected to achieve a consistent and effective absorption of NO in the body (see Reaction 5)).

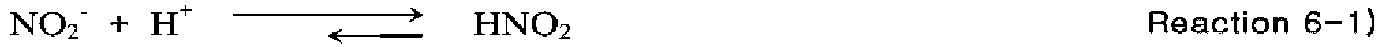

HNO₂ + R₂N-H → R₂N-NO + H₂O Reaction 6-2)
^{Footnote1)} In Reaction 6-1), the reverse reaction does not proceed well because the environment in stomach juice is strongly acidic
^{Footnote2)} In Reaction 6-2), secondary amines such as piperidine and pyrrolidine are found in fish, meat, cheese, and spices, while secondary amines such as dimethylamine and diethylamine are found in grains and tea

Various studies have been conducted to inhibit the formation of the potentially carcinogenic N-nitrosamines (R₂N-NO) from NO₂⁻ under the strongly acidic conditions of the stomach. Among these studies, one study in particular (Shenoy et al., Cancer Lett. 1992; 65(3): 227-232) demonstrated that polyphenols (hereinafter referred to as PhOH) present in food, cysteine (hereinafter referred to as Cys), and glutathione (GSH) containing cysteine can preferentially react with NO₂⁻ over secondary amines (R₂N), thereby blocking the formation of R₂N-NO (see Reactions 7-2) and 7-3)).

NO₂⁻ + R₂NH + H⁺ → R₂N-NO + H₂O Reaction 7-1)

NO₂⁻ + R₂NH + PhOH + H⁺ → PhO-NO + R₂NH + H₂O Reaction 7-2)

NO₂⁻ + R₂NH + GSH + H⁺ → GSNO + R₂NH + H₂O Reaction 7-3)

^{Footnote1)} All reactions shown above are observed only under strong acidic conditions such as those in the stomach
^{Footnote2)} In Reaction 7-2), PhOH reacts more effectively with NO₂⁻ than R₂NH, producing mainly PhO-N=O
^{Footnote3)} In Reaction 7-3), GSH reacts more effectively with NO₂⁻ than R₂NH, producing mainly GSNO
^{Footnote4)} NO cannot be produced from NO₂⁻ due to the above-mentioned reactions

Reactions 7-2) and 7-3) may have the advantage of blocking the formation of N-nitrosamines; however, they may also have the disadvantage of inhibiting NO generation from ingested NO₂⁻, thereby reducing the effective absorption of NO in the body. Nevertheless, if PhO-NO observed in Reaction 7-2) or GSNO observed in Reaction 7-3) generates NO in the intestinal environment, such as the small and large intestines, absorption of NO into the body becomes possible. In the case of GSNO formed in the stomach and reaching the intestines, it can react with the abundantly present GSH in the small intestine to generate NO (see Reaction 8)). Considering Reactions 7-3) and 8-1), when GSH is co-ingested with NO₂⁻, N-nitrosamine formation in the stomach is blocked, and NO is generated in the intestines, ultimately allowing effective absorption of NO into the body. 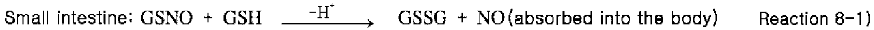 Small intestine: GSNO + GSNO → GSSG + 2NO(absorbed into the body) Reaction 8-2)

In the body: 2NO + O₂ → 2NO₂⁻ Reaction 8-3)
^{Footnote1)} In Reaction 8-1), GSH is produced in the liver and secreted into the small intestine through the bile duct
^{Footnote2)} Reaction 8-2) is presumed to be a spontaneous reaction that may occur when the concentration of GSNO is high
^{Footnote3)} Because NO Is hydrophobic, It Is absorbed In the body, and due to Its high reactivity and short lifetime, it reacts with oxygen and is oxidized to NO₂⁻
^{Footnote4)}Oxidized NO₂⁻ is hydrophilic, so it can be transported to various parts of the body through the bloodstream

In the human body, NO metabolites are reduced to NO under hypoxic or acidic conditions, leading to vasodilation or promotion of capillary formation, thereby regulating blood circulation (Lundberg et al., Nat Rev Drug Discov. 2008, 7: 156-167). The four major physiological functions of blood circulation can be considered as: (1) supply of oxygen and nutrients, (2) removal of carbon dioxide and waste products, (3) maintenance of body temperature, and (4) immune function. Therefore, NO can influence nutrient absorption, affect waste excretion, and regulate body temperature and immune function. For example, NO can normalize high blood pressure by dilating blood vessels and regulating blood flow (Amaral et al., Redox Biol. 2015, 5: 340-346). Consequently, NO metabolites that can be converted to NO may be developed as antihypertensive agents with minimal side effects. Additionally, several recent studies have reported that NO metabolites exhibit blood glucose-lowering effects (Khalifi et al., Nitric Oxide. 2015, 44: 24-30), promote insulin secretion from beta cells (Nystrom et al., Free Radic Biol Med. 2012, 53: 1017-1023), improve insulin resistance (Ohtake et al., Nitric Oxide. 2015, 44: 31-38), and ameliorate diabetic complications (Bahadoran et al., Nutr Metab (Lond). 2015, 12: 16. doi: 10.1186/s12986-015-0013-6). Accordingly, various studies are underway to develop NO metabolites as therapeutics for diabetes and its complications (Ghasemi et al., Nitric Oxide. 2017, 70: 9-24). However, when NO metabolites are orally administered for the treatment of various diseases, the three aforementioned health-hazardous issues may arise.

Meanwhile, Korean Patent Registration No. 2256793 discloses a "Method for preparing natural fermented composition containing fixed nitric oxide precursor and natural fermented composition prepared by this method," and Korean Patent Publication No. 2019-0084592 discloses a "Method for fixing nitric oxide in fermented natural product and natural fermented composition prepared by this method." However, neither disclosure provides any description regarding the "Composition for inhibiting formation of carcinogenic N-nitrosamines in the stomach and use thereof" of the present invention.

### DETAILED DESCRIPTION

### TECHNICAL PROBLEMS TO BE SOLVED

The present invention is devised in view of the above-mentioned needs. Inventors of the present invention orally administered a mixture in which glutathione, caffeic acid, chlorogenic acid, coffee extract, *Dendropanax morbifera* extract, or vitamin B12 (cyanocobalamin) is combined with a fermented natural product in which nitric oxide metabolites are fixed and stabilized, to experimental animal models of hypertension, enteritis, stress-induced bowel syndrome, arthritis, liver disease, and atopic dermatitis. As a result, compared to experimental groups administered only with the fermented natural product, the groups administered with the mixture of glutathione, caffeic acid, chlorogenic acid, coffee extract, *Dendropanax morbifera* extract, or vitamin B12 showed superior improvements in hypertension, enteritis, stress-induced bowel syndrome, arthritis, liver disease, and atopic dermatitis, thereby the present invention is completed.

### TECHNICAL MEANS FOR SOLVING PROBLEMS

To solve the problems that are described in the above, the present invention provides a composition for preventing, ameliorating, or treating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.

### ADVANTAGEOUS EFFECT OF THE INVENTION

The composition of the present invention is a mixture of nitric oxide metabolites with glutathione, caffeic acid, chlorogenic acid, coffee extract, *Dendropanax morbifera* extract, or vitamin B12, designed to block the formation of carcinogenic N-nitrosamines in the stomach juice (strongly acidic conditions) and to generate a certain amount of nitric oxide in the small and large intestines, thereby enabling absorption of nitric oxide metabolites in the body. It is expected to be widely applicable in the relevant fields for the treatment and symptom improvement of diseases known to be ameliorated by nitric oxide, such as hypertension, enteritis, arthritis, liver disease, and atopic dermatitis.

### BRIEF DESCRIPTION OF DRAWINGS

Figure 1 is a graph analyzing the disease activity index in an ulcerative colitis model following the administration of the fermented liquid of natural product and glutathione samples. *: p < 0.05 (compared to the control group).
Figure 2 is a graph analyzing the disease activity index in an ulcerative colitis model following the administration of the fermented liquid of natural product and caffeic acid samples. *: p < 0.05 (compared to the control group).
Figure 3 is a graph analyzing the disease activity index in an ulcerative colitis model following the administration of the fermented liquid of natural product and chlorogenic acid samples. *: p < 0.05 (compared to the control group).
Figure 4 is a graph analyzing the disease activity index in an ulcerative colitis model following the administration of the fermented liquid of natural product and coffee extract samples. *: p < 0.05 (compared to the control group).
Figure 5 is a graph analyzing the disease activity index in an ulcerative colitis model following the administration of the fermented liquid of natural product and *Dendropanax morbifera* extract samples. *: p < 0.05 (compared to the control group).
Figure 6 is a graph analyzing the disease activity index in an ulcerative colitis model following the administration of the fermented liquid of natural product and vitamin B12 (cyanocobalamin) samples. *: p < 0.05 (compared to the control group).
Figure 7 is a graph analyzing changes in colon length in an ulcerative colitis model following the administration of the fermented liquid of natural product and glutathione samples. *: p < 0.05 (compared to the control group).
Figure 8 is a graph analyzing changes in colon length in an ulcerative colitis model following the administration of the fermented liquid of natural product and caffeic acid samples. *: p < 0.05 (compared to the control group).
Figure 9 is a graph analyzing changes in colon length in an ulcerative colitis model following the administration of the fermented liquid of natural product and chlorogenic acid samples. *: p < 0.05 (compared to the control group).
Figure 10 is a graph analyzing changes in colon length in an ulcerative colitis model following the administration of the fermented liquid of natural product and coffee extract samples. *: p < 0.05 (compared to the control group).
Figure 11 is a graph analyzing changes in colon length in an ulcerative colitis model following the administration of the fermented liquid of natural product and *Dendropanax morbifera* extract samples. *: p < 0.05 (compared to the control group).
Figure 12 is a graph analyzing changes in colon length in an ulcerative colitis model following the administration of the fermented liquid of natural product and vitamin B12 samples. *: p < 0.05 (compared to the control group).
Figure 13 is a graph analyzing serum levels of TNF-α (a) and IL-1β (b) in an ulcerative colitis model following the administration of the fermented liquid of natural product and glutathione samples. *: p < 0.05 (compared to the control group).
Figure 14 is a graph analyzing serum levels of TNF-α (a) and IL-1β (b) in an ulcerative colitis model following the administration of the fermented liquid of natural product and caffeic acid samples. *: p < 0.05 (compared to the control group).
Figure 15 is a graph analyzing serum levels of TNF-α (a) and IL-1β (b) in an ulcerative colitis model following the administration of the fermented liquid of natural product and chlorogenic acid samples. *: p < 0.05 (compared to the control group).
Figure 16 is a graph analyzing serum levels of TNF-α (a) and IL-1β (b) in an ulcerative colitis model following the administration of the fermented liquid of natural product and coffee extract samples. *: p < 0.05 (compared to the control group).
Figure 17 is a graph analyzing serum levels of TNF-α (a) and IL-1β (b) in an ulcerative colitis model following the administration of the fermented liquid of natural product and *Dendropanax morbifera* extract samples. *: p < 0.05 (compared to the control group).
Figure 18 is a graph analyzing serum levels of TNF-α (a) and IL-1β (b) in an ulcerative colitis model following the administration of the fermented liquid of natural product and vitamin B12 samples. *: p < 0.05 (compared to the control group).
Figure 19 is a schematic diagram of an experiment to determine the improvement effect on stress-induced bowel syndrome. WAS refers to water avoidance stress.
Figure 20 shows the results of measuring the number of fecal pellets in each experimental group in a stress-induced bowel syndrome model following the administration of the fermented liquid of natural product and glutathione samples, in which (a) represents the daily average number of fecal pellets and (b) represents the cumulative number of fecal pellets until the end of the experiment.
Figure 21 shows the results of measuring the number of fecal pellets in each experimental group in a stress-induced bowel syndrome model following the administration of the fermented liquid of natural product and caffeic acid samples, in which (a) represents the daily average number of fecal pellets and (b) represents the cumulative number of fecal pellets until the end of the experiment.
Figure 22 shows the results of measuring the number of fecal pellets in each experimental group in a stress-induced bowel syndrome model following the administration of the fermented liquid of natural product and chlorogenic acid samples, in which (a) represents the daily average number of fecal pellets and (b) represents the cumulative number of fecal pellets until the end of the experiment.
Figure 23 shows the results of measuring the number of fecal pellets in each experimental group in a stress-induced bowel syndrome model following the administration of the fermented liquid of natural product and coffee extract samples, in which (a) represents the daily average number of fecal pellets and (b) represents the cumulative number of fecal pellets until the end of the experiment.
Figure 24 shows the results of measuring the number of fecal pellets in each experimental group in a stress-induced bowel syndrome model following the administration of the fermented liquid of natural product and *Dendropanax morbifera* extract samples, in which (a) represents the daily average number of fecal pellets and (b) represents the cumulative number of fecal pellets until the end of the experiment.
Figure 25 shows the results of measuring the number of fecal pellets in each experimental group in a stress-induced bowel syndrome model following the administration of the fermented liquid of natural product and vitamin B12 samples, in which (a) represents the daily average number of fecal pellets and (b) represents the cumulative number of fecal pellets until the end of the experiment.
Figure 26 shows the results of measuring the changes in blood pressure before and after administration of the fermented liquid of natural product and glutathione samples in a hypertension model.
Figure 27 shows the results of measuring the changes in blood pressure before and after administration of the fermented liquid of natural product and caffeic acid samples in a hypertension model.
Figure 28 shows the results of measuring the changes in blood pressure before and after administration of the fermented liquid of natural product and chlorogenic acid samples in a hypertension model.
Figure 29 shows the results of measuring the changes in blood pressure before and after administration of the fermented liquid of natural product and coffee extract samples in a hypertension model.
Figure 30 shows the results of measuring the changes in blood pressure before and after administration of the fermented liquid of natural product and *Dendropanax morbifera* extract samples in a hypertension model.
Figure 31 shows the results of measuring the changes in blood pressure before and after administration of the fermented liquid of natural product and vitamin B12 samples in a hypertension model.
Figure 32 shows the results of measuring the changes in joint swelling thickness after administration of the fermented liquid of natural product and glutathione samples in an arthritis induction model using MIA (monosodium iodoacetate). *: p < 0.05 (compared to the control group).
Figure 33 shows the results of measuring the changes in joint swelling thickness after administration of the fermented liquid of natural product and caffeic acid samples in an arthritis induction model using MIA. *: p < 0.05 (compared to the control group).
Figure 34 shows the results of measuring the changes in joint swelling thickness after administration of the fermented liquid of natural product and chlorogenic acid samples in an arthritis induction model using MIA. *: p < 0.05 (compared to the control group).
Figure 35 shows the results of measuring the changes in joint swelling thickness after administration of the fermented liquid of natural product and coffee extract samples in an arthritis induction model using MIA. *: p < 0.05 (compared to the control group).
Figure 36 shows the results of measuring the changes in joint swelling thickness after administration of the fermented liquid of natural product and *Dendropanax morbifera* extract samples in an arthritis induction model using MIA. *: p < 0.05 (compared to the control group).
Figure 37 shows the results of measuring the changes in joint swelling thickness after administration of the fermented liquid of natural product and vitamin B12 samples in an arthritis induction model using MIA. *: p < 0.05 (compared to the control group).
Figure 38 shows the arthritis index evaluated after administration of the fermented liquid of natural product and glutathione samples in an arthritis induction model using MIA. *: p < 0.05 (compared to the control group).
Figure 39 shows the arthritis index evaluated after administration of the fermented liquid of natural product and caffeic acid samples in an arthritis induction model using MIA. *: p < 0.05 (compared to the control group).
Figure 40 shows the arthritis index evaluated after administration of the fermented liquid of natural product and chlorogenic acid samples in an arthritis induction model using MIA. *: p < 0.05 (compared to the control group).
Figure 41 shows the arthritis index evaluated after administration of the fermented liquid of natural product and coffee extract samples in an arthritis induction model using MIA. *: p < 0.05 (compared to the control group).
Figure 42 shows the arthritis index evaluated after administration of the fermented liquid of natural product and *Dendropanax morbifera* extract samples in an arthritis induction model using MIA. *: p < 0.05 (compared to the control group).
Figure 43 shows the arthritis index evaluated after administration of the fermented liquid of natural product and vitamin B12 samples in an arthritis induction model using MIA. *: p < 0.05 (compared to the control group).
Figure 44 is a graph analyzing serum levels of TNF-α (a), Metallopeptidase-9 (b), and Prostaglandin E2 (PGE₂) (c) after administration of the fermented liquid of natural product and glutathione samples in an arthritis induction model using MIA.
Figure 45 is a graph analyzing serum levels of TNF-α (a), Metallopeptidase-9 (b), and PGE₂ (c) after administration of the fermented liquid of natural product and caffeic acid samples in an arthritis induction model using MIA.
Figure 46 is a graph analyzing serum levels of TNF-α (a), Metallopeptidase-9 (b), and PGE₂ (c) after administration of the fermented liquid of natural product and chlorogenic acid samples in an arthritis induction model using MIA.
Figure 47 is a graph analyzing serum levels of TNF-α (a), Metallopeptidase-9 (b), and PGE₂ (c) after administration of the fermented liquid of natural product and coffee extract samples in an arthritis induction model using MIA.
Figure 48 is a graph analyzing serum levels of TNF-α (a), Metallopeptidase-9 (b), and PGE₂ (c) after administration of the fermented liquid of natural product and *Dendropanax morbifera* extract samples in an arthritis induction model using MIA.
Figure 49 is a graph analyzing serum levels of TNF-α (a), Metallopeptidase-9 (b), and PGE₂ (c) after administration of the fermented liquid of natural product and vitamin B12 samples in an arthritis induction model using MIA.
Figure 50 shows the results of measuring serum ALT and AST levels after administration of the fermented liquid of natural product and glutathione samples in an acute liver injury model. *: p < 0.05 (compared to the control group).
Figure 51 shows the results of measuring serum ALT and AST levels after administration of the fermented liquid of natural product and caffeic acid samples in an acute liver injury model. *: p < 0.05 (compared to the control group).
Figure 52 shows the results of measuring serum ALT and AST levels after administration of the fermented liquid of natural product and chlorogenic acid samples in an acute liver injury model. *: p < 0.05 (compared to the control group).
Figure 53 shows the results of measuring serum ALT and AST levels after administration of the fermented liquid of natural product and coffee extract samples in an acute liver injury model. *: p < 0.05 (compared to the control group).
Figure 54 shows the results of measuring serum ALT and AST levels after administration of the fermented liquid of natural product and *Dendropanax morbifera* extract samples in an acute liver injury model. *: p < 0.05 (compared to the control group).
Figure 55 shows the results of measuring serum ALT and AST levels after administration of the fermented liquid of natural product and vitamin B12 samples in an acute liver injury model. *: p < 0.05 (compared to the control group).
Figure 56 shows the results of measuring morphological changes in the right ear after administration of the fermented liquid of natural product and glutathione samples in an atopic dermatitis model. *: p < 0.05 (compared to the control group).
Figure 57 shows the results of measuring morphological changes in the right ear after administration of the fermented liquid of natural product and caffeic acid samples in an atopic dermatitis model. *: p < 0.05 (compared to the control group).
Figure 58 shows the results of measuring morphological changes in the right ear after administration of the fermented liquid of natural product and chlorogenic acid samples in an atopic dermatitis model. *: p < 0.05 (compared to the control group).
Figure 59 shows the results of measuring morphological changes in the right ear after administration of the fermented liquid of natural product and coffee extract samples in an atopic dermatitis model. *: p < 0.05 (compared to the control group).
Figure 60 shows the results of measuring morphological changes in the right ear after administration of the fermented liquid of natural product and *Dendropanax morbifera* extract samples in an atopic dermatitis model. *: p < 0.05 (compared to the control group).
Figure 61 shows the results of measuring morphological changes in the right ear after administration of the fermented liquid of natural product and vitamin B12 samples in an atopic dermatitis model. *: p < 0.05 (compared to the control group).
Figure 62 shows the results of measuring the thickness changes of the right ear after administration of the fermented liquid of natural product and glutathione samples in an atopic dermatitis model.
Figure 63 shows the results of measuring the thickness changes of the right ear after administration of the fermented liquid of natural product and caffeic acid samples in an atopic dermatitis model.
Figure 64 shows the results of measuring the thickness changes of the right ear after administration of the fermented liquid of natural product and chlorogenic acid samples in an atopic dermatitis model.
Figure 65 shows the results of measuring the thickness changes of the right ear after administration of the fermented liquid of natural product and coffee extract samples in an atopic dermatitis model.
Figure 66 shows the results of measuring the thickness changes of the right ear after administration of the fermented liquid of natural product and *Dendropanax morbifera* extract samples in an atopic dermatitis model.
Figure 67 shows the results of measuring the thickness changes of the right ear after administration of the fermented liquid of natural product and vitamin B12 samples in an atopic dermatitis model.
Figure 68 shows the results of measuring the spleen index at the end of the experiment for each group after administration of the fermented liquid of natural product and glutathione samples in an atopic dermatitis model. *: p < 0.05 (compared to the control group).
Figure 69 shows the results of measuring the spleen index at the end of the experiment for each group after administration of the fermented liquid of natural product and caffeic acid samples in an atopic dermatitis model. *: p < 0.05 (compared to the control group).
Figure 70 shows the results of measuring the spleen index at the end of the experiment for each group after administration of the fermented liquid of natural product and chlorogenic acid samples in an atopic dermatitis model. *: p < 0.05 (compared to the control group).
Figure 71 shows the results of measuring the spleen index at the end of the experiment for each group after administration of the fermented liquid of natural product and coffee extract samples in an atopic dermatitis model. *: p < 0.05 (compared to the control group).
Figure 72 shows the results of measuring the spleen index at the end of the experiment for each group after administration of the fermented liquid of natural product and *Dendropanax morbifera* extract samples in an atopic dermatitis model. *: p < 0.05 (compared to the control group).
Figure 73 shows the results of measuring the spleen index at the end of the experiment for each group after administration of the fermented liquid of natural product and vitamin B12 samples in an atopic dermatitis model. *: p < 0.05 (compared to the control group).
Figure 74 shows the results of measuring serum levels of TNF-α (a) and IL-4 (b) at the end of the experiment for each group after administration of the fermented liquid of natural product and glutathione samples in an atopic dermatitis model. *: p < 0.05 (compared to the control group).
Figure 75 shows the results of measuring serum levels of TNF-α (a) and IL-4 (b) at the end of the experiment for each group after administration of the fermented liquid of natural product and caffeic acid samples in an atopic dermatitis model. *: p < 0.05 (compared to the control group).
Figure 76 shows the results of measuring serum levels of TNF-α (a) and IL-4 (b) at the end of the experiment for each group after administration of the fermented liquid of natural product and chlorogenic acid samples in an atopic dermatitis model. *: p < 0.05 (compared to the control group).
Figure 77 shows the results of measuring serum levels of TNF-α (a) and IL-4 (b) at the end of the experiment for each group after administration of the fermented liquid of natural product and coffee extract samples in an atopic dermatitis model. *: p < 0.05 (compared to the control group).
Figure 78 shows the results of measuring serum levels of TNF-α (a) and IL-4 (b) at the end of the experiment for each group after administration of the fermented liquid of natural product and *Dendropanax morbifera* extract samples in an atopic dermatitis model. *: p < 0.05 (compared to the control group).
Figure 79 shows the results of measuring serum levels of TNF-α (a) and IL-4 (b) at the end of the experiment for each group after administration of the fermented liquid of natural product and vitamin B12 samples in an atopic dermatitis model. *: p < 0.05 (compared to the control group).

### BEST MODE(S) FOR CARRYING OUT THE INVENTION

The present invention provides a pharmaceutical composition for preventing or treating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.

In the pharmaceutical composition of the present invention, the active ingredients may inhibit the formation of carcinogenic N-nitrosamines in the stomach and promote nitric oxide generation in the intestines.

The concentration of nitric oxide in the human body can be predicted as the total amount derived from the sum of nitrite ions (NO₂⁻) and nitrate ions (NO₃⁻); however, the actually utilizable nitric oxide concentration can be predicted based on the concentration of NO₂⁻ that can be converted into nitric oxide (NO) in the blood. As a nitric oxide metabolite, NO₂⁻ is a hydrophilic and relatively stable ionic substance, which can dissolve in blood and bodily fluids and move throughout the body, but cannot pass through organ barriers, vascular barriers, or cell membranes. However, if NO₂⁻ is reduced to lipophilic NO by nitrite reductase or under acidic conditions, it can cross organ barriers, vascular barriers, and cell membranes. Therefore, for NO₂⁻ to reach target cells and exert specific pharmacological effects or to be absorbed into the body from the stomach or intestines, it must first be reduced to NO.

In order to increase the low concentration of nitric oxide metabolites in the blood or to obtain the pharmacological effects of nitric oxide, nitric oxide metabolites (particularly NO₂⁻) may be administered orally. However, this approach may cause the following two health-related problems. First, NO₂⁻ administered orally can be rapidly absorbed as excessive NO under the highly acidic conditions of gastric juice, potentially leading to sudden decreases in blood pressure, hypoglycemia, and cyanosis. Second, under the highly acidic conditions of gastric juice, orally administered NO₂⁻ can react with secondary amines to form carcinogenic N-nitrosamines.

The pharmaceutical composition according to the present invention is developed as a composition in which a nitric oxide metabolites (NO₂⁻) contained in a fermented natural product react chemically under the highly acidic conditions of the stomach with any one of glutathione, caffeic acid, chlorogenic acid, vitamin B12, coffee extract, or *Dendropanax morbifera* extract to form S-nitrosoglutathione (GSNO), thereby blocking the formation of N-nitrosamines (R₂N-NO) and generating a certain amount of nitric oxide (NO) in the intestinal environment, enabling the absorption of nitric oxide metabolites into the body. In the present invention, "coffee extract" is used with the same meaning as coffee extract liquid, and "*Dendropanax morbifera* extract" refers to an extract from the *Dendropanax morbifera* tree.

In the pharmaceutical composition of the present invention, the fermented natural product in which the nitric oxide metabolites are fixed and stabilized may be prepared by a method comprising adding a fermentation strain to a nitrogen-containing natural product and carrying out fermentation at a temperature of 18 to 35°C and a dissolved oxygen concentration of 0.03 to 0.1 mg/L for 2 to 30 days; however, the present invention is not limited thereto. The nitrogen-containing natural product may include, but is not limited to, soybean, garlic, bean sprout, cabbage, or whey. A detailed method for preparing the fermented natural product according to the present invention can be found in Korean Patent Registration No. 2129038.

The fermented natural product according to the present invention may be fermented by adding 0.01 to 3 parts by weight of a fermentation strain based on 100 parts by weight of a mixture of a nitrogen-containing natural product and water. Preferably, the nitrogen-containing natural product and water are mixed at a weight ratio of 1:0.5 to 1, and 0.01 to 3 parts by weight of the fermentation strain are added based on 100 parts by weight of the resulting mixture for fermentation; however, the present invention is not limited thereto.

In addition, the fermentation may be carried out at a temperature of 10 to 40°C, preferably at 18 to 35°C, more preferably at 20 to 33°C, and even more preferably at 25 to 33°C; however, the present invention is not limited thereto.

In addition, the fermentation may be carried out under a dissolved oxygen concentration of 0.01 to 0.1 mg/L, preferably 0.03 to 0.1 mg/L; however, the present invention is not limited thereto.

In addition, the fermentation may be carried out for 2 to 30 days; however, the present invention is not limited thereto.

In addition, the fermentation may be terminated when the chemical oxygen demand reaches 200 to 700 mg/L; however, the present invention is not limited thereto.

In addition, the fermentation strain may preferably be *Bacillus subtilis* - Chun Hyun Su, having the deposit number KCTC12501BP; however, the present invention is not limited thereto.

In addition, in the pharmaceutical composition according to the present invention, the fermented natural product in which the nitric oxide metabolites are fixed and stabilized may preferably contain 0.1 to 10 mM based on NO₂⁻; however, the present invention is not limited thereto.

In this specification, the term "nitric oxide metabolites" collectively refers to nitrite (NO₂⁻), R-NO₂⁻, R-NO, RO-NO, nitrate (NO₃⁻), R-NO₃⁻, GS-NO (or GSNO), GS-NO₂, and the like, in which R- represents a carbon chain (e.g., polyphenols, fatty acids) from which one hydrogen atom has been removed, RO- represents a residue in which one hydrogen atom is removed from ROH, and GS- represents a residue in which one hydrogen atom is removed from the cysteine moiety of glutathione (GSH). That is, it refers to a functional group capable of binding nitric oxide or nitrite ions. The nitric oxide metabolites are converted into nitric oxide under acidic and reducing conditions. Under strongly acidic conditions such as in the stomach, the nitric oxide metabolites are reduced by hydrogen ions (H⁺) to release nitric oxide, whereas under mildly acidic conditions such as in the intestines (small and large intestines), the nitric oxide metabolites can be reduced to nitric oxide by reducing agents such as polyphenols, glutathione, vitamin C, or bacterial nitric oxide reductases.

In particular, GSNO, known as a nitric oxide metabolite or nitric oxide adduct, can safely generate nitric oxide by reacting with glutathione (GSH) secreted from the liver via the bile duct or with GSH secreted by intestinal bacteria (see Reaction 8-1)). The nitric oxide (NO) thus generated in the intestinal environment penetrates into the body and is oxidized into the nitric oxide metabolite (NO₂⁻), thereby reaching the body fluids or blood vessels (see Reaction 8-3)).

In the pharmaceutical composition according to the present invention, the glutathione may be in the form of pure glutathione or an extract containing glutathione, but is not limited thereto. "Glutathione (GSH)" is a small peptide composed of three amino acids - glutamate, cysteine, and glycine - and is an endogenous reducing agent produced in plants, animals, fungi, and certain bacteria and archaea. In the body or within cells, glutathione binds to free radicals, peroxides, toxic substances, and heavy metals to prevent cellular damage. In addition, GSH can react with nitric oxide (NO) to form S-nitrosoglutathione (GSNO). GSNO generated through this process delivers NO to where it is needed via the vasculature, and intracellularly, it stores NO for a certain period, releasing it as NO when required to exert the pharmacological effects of NO.

In the pharmaceutical composition according to the present invention, the caffeic acid or chlorogenic acid, as phenolic compounds, promote the reduction of nitric oxide metabolites in the fermented natural product to nitric oxide. The coffee extract is an extract known to be rich in caffeic acid and chlorogenic acid, and the *Dendropanax morbifera* extract also contains various polyphenolic compounds. In addition, the vitamin B12 compound used in the present invention, cyanocobalamin, is reduced in the body from cobalt in the +3 oxidation state to the +2 or +1 state, and is then converted to adenosylcobalamin or methylcobalamin to function as a coenzyme.

The pharmaceutical composition of the present invention may further include a suitable carrier, excipient, or diluent commonly used in the preparation of pharmaceutical compositions.

In addition, the pharmaceutical composition of the present invention may be formulated for use in oral dosage forms such as granules, tablets, or capsules according to conventional methods, but is not limited thereto.

Carriers, excipients, and diluents that may be included in the pharmaceutical composition of the present invention include a variety of compounds or mixtures such as lactose, dextrose, sucrose, sorbitol, mannitol, xylitol, erythritol, maltitol, starch, acacia gum, alginate, gelatin, calcium phosphate, calcium silicate, cellulose, methylcellulose, amorphous cellulose, polyvinylpyrrolidone, water, methylparaben, propylparaben, talc, magnesium stearate, and mineral oil. When formulating, commonly used fillers, bulking agents, binders, humectants, disintegrants, and surfactants can be employed as diluents or excipients. Oral solid formulations may include tablets, pills, powders, granules, and capsules, which are prepared by mixing the fermented extract with one or more excipients such as starch, calcium carbonate, sucrose or lactose, and gelatin. In addition to basic excipients, lubricants like magnesium stearate or talc may also be used. Oral liquid formulations include suspensions, solutions, emulsions, and syrups, which may contain simple diluents such as water or liquid paraffin, as well as various excipients including humectants, sweeteners, flavoring agents, and preservatives. Parenteral formulations may include sterile aqueous solutions, non-aqueous solvents, suspensions, emulsions, lyophilized preparations, and suppositories. Non-aqueous solvents or suspensions may use agents such as propylene glycol, polyethylene glycol, vegetable oils like olive oil, or injectable esters such as ethyl oleate. Suppository bases may include Witepsol, macrogol, Tween 61, cocoa butter, laurin butter, or glycerogelatin.

The pharmaceutical composition of the present invention can be administered to mammals such as rats, mice, livestock, or humans via various routes, and all modes of administration are conceivable; preferably, it may be administered orally or rectally, but is not limited thereto.

The pharmaceutical composition of the present invention may be administered as a standalone therapeutic agent or in combination with other therapeutics, and may be administered sequentially or simultaneously with conventional treatments, either as a single or multiple dose. Considering all the above factors, it is important to administer an amount that achieves maximum efficacy with minimal dosage and without side effects, which can be readily determined by those skilled in the art.

Specifically, the pharmaceutically effective amount of the pharmaceutical composition according to the present invention may vary depending on the patient's age, sex, and body weight. In general, it can be administered at 2 mg to 4 mg per kg of body weight, preferably 3 mg, daily or every other day, and may be divided into 1 to 3 doses per day. However, the dosage may be increased or decreased depending on the route of administration, severity of the disease, sex, body weight, and age; therefore, the above dosage does not in any way limit the scope of the present invention.

The present invention also provides a functional health food composition for preventing or ameliorating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.

In the functional health food composition according to the present invention, the fermented natural product in which the nitric oxide metabolites are fixed and stabilized, as well as glutathione, caffeic acid, chlorogenic acid, vitamin B12, coffee extract, and *Dendropanax morbifera* extract, are as described in the above.

There is no particular limitation on the type of the functional health food. Examples of the functional health food containing the functional health food composition include meat, sausages, bread, chocolate, candies, snacks, cookies, pizza, ramen, other noodles, gum, rice cakes, dairy products including ice cream, various soups, beverages, tea, drink products, alcoholic beverages, and vitamin complexes, and encompass all functional health foods in the conventional sense.

The functional health food may be prepared in any one formulation selected from powders, granules, pills, tablets, capsules, candies, syrups, effervescent tablets, and beverages, but is not limited thereto.

When the functional health food composition of the present invention is used as a food additive, the composition may be added directly or used together with other foods or food ingredients, and can be appropriately applied according to conventional methods. The active ingredients may be used in amounts suitable for their intended purpose (prevention or amelioration). Generally, when manufacturing foods or beverages, the composition of the present invention is added in an amount of 15 parts by weight or less, preferably 10 parts by weight or less, relative to the raw materials. However, for long-term intake for health management purposes, it may be used in amounts within a range that poses no safety concerns.

The functional health food of the present invention may include ingredients commonly added in food production, such as proteins, carbohydrates, fats, nutrients, and seasonings. For example, when prepared as a drink, in addition to the active ingredients, natural carbohydrates or flavoring agents may be included as additional components. The natural carbohydrates are preferably monosaccharides (e.g., glucose, fructose), disaccharides (e.g., maltose, sucrose), oligosaccharides, polysaccharides (e.g., dextrin, cyclodextrin), or sugar alcohols (e.g., xylitol, sorbitol, erythritol). The flavoring agents may include natural flavors (e.g., thaumatin, stevia extract) and synthetic flavors (e.g., saccharin, aspartame). In addition to the functional health food composition, various supplements, vitamins, electrolytes, flavor enhancers, colorants, pectins and their salts, alginates and their salts, organic acids, protective colloid thickeners, pH adjusters, stabilizers, preservatives, glycerin, alcohols, and carbonating agents used in carbonated beverages may also be included.

The present invention also provides a veterinary composition for preventing or treating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.

In the veterinary composition according to the present invention, the fermented natural product in which the nitric oxide metabolites are fixed and stabilized, as well as glutathione, caffeic acid, chlorogenic acid, vitamin B12, coffee extract, and *Dendropanax morbifera* extract, are as described in the above.

The veterinary composition of the present invention may further include suitable excipients and diluents commonly used in conventional methods. Examples of the excipients and diluents that can be included in the veterinary composition of the present invention include lactose, dextrose, sucrose, sorbitol, mannitol, xylitol, erythritol, maltitol, starch, acacia gum, alginate, gelatin, calcium phosphate, calcium silicate, cellulose, methylcellulose, amorphous cellulose, polyvinylpyrrolidone, water, methyl hydroxybenzoate, propyl hydroxybenzoate, talc, magnesium stearate, cetanol, stearyl alcohol, liquid paraffin, sorbitan monostearate, polysorbate 60, methylparaben, propylparaben, and mineral oil.

The veterinary composition according to the present invention may further include fillers, anti-caking agents, lubricants, humectants, flavoring agents, emulsifiers, preservatives, and the like. The veterinary composition can be formulated using methods well known in the art so as to provide rapid, sustained, or delayed release of the active ingredients after administration to animals. The formulations may be in the form of powders, granules, tablets, capsules, suspensions, emulsions, solutions, syrups, aerosols, soft or hard gelatin capsules, suppositories, sterile injectable solutions, or sterile topical preparations.

The effective amount of the veterinary composition according to the present invention can be appropriately selected depending on the individual animal. It may be determined based on factors well known in physiology and veterinary medicine, including the severity of the disease or condition, the age, weight, health status, or sex of the animal, sensitivity to the active ingredients of the present invention, the route and duration of administration, and any other compositions administered in combination or concurrently with the composition.

In this specification, the term "fermented liquid" is used interchangeably to refer to a "fermented natural product in which nitric oxide metabolites are fixed and stabilized."

Hereinbelow, the present invention is explained in greater detail in view of the Examples. However, it is evident that the following Examples are given only for exemplification of the present invention and by no means the present invention is limited to the following Examples.

### EXAMPLES

### 1. Experimental Methods

### 1-1) Preparation of reagents and test samples

The fermented liquids prepared from lettuce, soybeans, garlic, bean sprouts, cabbage, or whey by HumanEnos Co., Ltd. containing 1 mM NO₂⁻ were used in the experiments. As a control for the fermented natural product, 1 mM sodium nitrite (NaNO₂) was used. Caffeic acid (CAS No. 331-39-5), chlorogenic acid (CAS No. 327-97-9), vitamin B12 (cyanocobalamin, CAS No. 68-19-9), and glutathione (CAS No. 70-18-8) were purchased from Sigma-Aldrich, prepared at a concentration of 1 mM, and mixed with the fermented liquid of natural product or sodium nitrite at a 1:1 weight ratio for use. Additionally, glutathione-containing dried yeast was purchased from Bision BioChem Co., Ltd. and used at 1 mM based on the glutathione content. Coffee extract was prepared by adding 20 L of purified water to 500 g of ground coffee beans, extracting at 94°C for 10 minutes, and then centrifuging at 4,500 rpm for 5 minutes to collect the supernatant for the experiments. *Dendropanax morbifera* extract was prepared by adding 4 L of fermented ethanol to 200 g of crushed *Dendropanax morbifera* tree stems and leaves, extracting at 88°C for 2 hours, and then centrifuging at 4,500 rpm for 5 minutes to obtain the supernatant for use. The coffee extract and *Dendropanax morbifera* extract were mixed with the fermented liquid of natural product at a 1:1 weight ratio for the experiments.

The fermented liquids of lettuce, soybeans, garlic, bean sprouts, cabbage, or whey refer to concentrated extracts of the fermented products prepared by the method disclosed in Korean Patent Registration No. 2129038. Briefly, each raw material was mixed with water at a weight ratio of 1:0.5-1, and 0.01-3 parts by weight of a fermentation strain was added based on 100 parts by weight of the mixture. The mixture was then fermented at 18-35°C under a dissolved oxygen concentration of 0.03 to 0.1 mg/L for 2 to 30 days. After fermentation, the mixture was centrifuged to separate solids, and the resulting supernatant was concentrated by stirring at 100-300 rpm at 20-30°C for 24 to 48 hours.

### 1-2) Measurement of nitric oxide metabolites concentration

The total amount of nitric oxide metabolites contained in the fermented liquid (meaning the combined concentration of NO₂⁻ and NO₃⁻, hereinafter referred to as NOₓ) was determined by adding nitrate reductase to reduce all NO₃⁻ present in the concentrated extract to NO₂⁻, and then measuring the total NO₂⁻ concentration to determine the total content or concentration of nitric oxide metabolites.

More specifically, for the fermented liquid, the total NO₂⁻ content was determined as follows: first, nitrate reductase was added to the fermented liquid and allowed to react for 1 hour for reduction. The reduced liquid was then diluted 1,000-fold with distilled water and centrifuged at 3,000 rpm for 3 minutes to remove insoluble polyphenols and dietary fiber. The resulting clear supernatant (50 µL) was taken as the measurement sample. Next, 50 µL of a NO₂⁻ standard solution and 50 µL of the measurement sample were each dispensed into a 96-well plate, followed by the addition of 100 µL of the test solution (a mixture of 1% sulfanilamide in 30% acetic acid and 0.1% N-(1-naphthyl)ethylenediamine in 60% acetic acid at a 1:2 ratio). The plate was left at room temperature for 10 minutes to induce the colorimetric reaction. Absorbance values for each well were then measured at 570 nm using a multi-plate reader, and the NOₓ concentration was calculated by comparing the absorbance of the sample with that of the standard solution.

### 2. Statistical Analysis

The results obtained from each experiment were expressed as mean±standard deviation (SD). Significance between two experimental groups was analyzed using a t-test, and for comparisons involving two or more groups, one-way analysis of variance (one-way ANOVA) was performed followed by Duncan's post hoc test. Differences were considered statistically significant when the p-value was not higher than 0.05 (p < 0.05).

### Example 1. Effect of mixture sample of fermented liquid containing nitric oxide metabolites and glutathione, caffeic acid, chlorogenic acid, coffee extract, Dendropanax morbifera extract, or vitamin B12 on amelioration of colitis

In this experiment, 6-week-old male Balb/c mice were purchased from Samtako (Korea) and acclimated for one week before use. The average weight of the Balb/c mice was 20-24 g, and the samples were administered orally once daily for three weeks. The animal housing room was maintained with a 12-hour light/dark cycle, a temperature of 23±2°C, and a humidity of 50-60%.

### 1-1. Group configuration and induction of ulcerative colitis

The experimental animals were classified as shown in the following Tables 1 and 2. The experimental group receiving a mixture of chlorogenic acid, vitamin B12, coffee extract, and *Dendropanax morbifera* extract was composed identically to the caffeic acid mixture group in Table 2. As described above, the coffee extract and *Dendropanax morbifera* extract were mixed with the fermented liquid of natural product in the same weight ratio, and a dose of 1 mM refers to the NO₂⁻ concentration in the fermented liquid of natural product.

Ulcerative colitis was induced in all experimental groups except the normal control by freely providing 3.0% DSS (dextran sulfate sodium) for five days. The number of animals per group was determined according to the 3R principle, using the minimum number sufficient for statistical significance.

**[Table 1]**

| Configuration of ulcerative colitis experimental groups using mixture of fermented liquid of natural product and glutathione (GSH) | | | | | |
|---|---|---|---|---|---|
| | Group | DSS | Material | Dose | n |
| 1 | Normal group | No | distilled water | | 5 |
| 2 | Control group | Yes | distilled water | | 5 |
| 3 | NaNO₂ | Yes | sodium nitrite | 1 mM | 5 |
| 4 | NaNO₂+50% GSH | Yes | sodium nitrite + Yeast extract 50% glutathione | 1 mM | 5 |
| 5 | NaNO₂+98% GSH | Yes | sodium nitrite + 98% glutathione | 1 mM | 5 |
| 6 | Fermented lettuce liquid | Yes | *Lactuca sativa* fermented extract | 1 mM | 5 |
| 7 | Fermented lettuce liquid+50% GSH | Yes | *Lactuca sativa* fermented extract + Yeast extract 50% glutathione | 1 mM | 5 |
| 8 | Fermented lettuce liquid+98% GSH | Yes | *Lactuca sativa* fermented extract + 98% glutathione | 1 mM | 5 |
| 9 | Fermented soybean liquid | Yes | bean fermented extract | 1 mM | 5 |
| 10 | Fermented soybean liquid+50% GSH | Yes | bean fermented extract + Yeast extract 50% glutathione | 1 mM | 5 |
| 11 | Fermented soybean liquid+98% GSH | Yes | bean fermented extract + 98% glutathione | 1 mM | 5 |
| 12 | Fermented garlic liquid | Yes | Garlic fermented extract | 1 mM | 5 |
| 13 | Fermented garlic liquid+50% GSH | Yes | Garlic fermented extract + Yeast extract 50% glutathione | 1 mM | 5 |
| 14 | Fermented garlic liquid+98% GSH | Yes | Garlic fermented extract + 98% glutathione | 1 mM | 5 |
| 15 | Fermented bean sprout liquid | Yes | bean sprout fermented extract | 1 mM | 5 |
| 16 | Fermented bean sprout liquid+50% GSH | Yes | bean sprout fermented extract + Yeast extract 50% glutathione | 1 mM | 5 |
| 17 | Fermented bean sprout liquid+98% GSH | Yes | bean sprout fermented extract + 98% glutathione | 1 mM | 5 |
| 18 | Fermented cabbage liquid | Yes | Cabbage fermented extract | 1 mM | 5 |
| 19 | Fermented cabbage liquid+50% GSH | Yes | Cabbage fermented extract + Yeast extract 50% glutathione | 1 mM | 5 |
| 20 | Fermented cabbage liquid+98% GSH | Yes | Cabbage fermented extract + 98% glutathione | 1 mM | 5 |
| 21 | Fermented whey liquid | Yes | Whey fermented extract | 1 mM | 5 |
| 22 | Fermented whey liquid+50% GSH | Yes | Whey fermented extract + Yeast extract 50% glutathione | 1 mM | 5 |
| 23 | Fermented whey liquid+98% GSH | Yes | Whey fermented extract + 98% glutathione | 1 mM | 5 |

**[Table 2]**

| Configuration of ulcerative colitis experimental groups using mixture of fermented liquid of natural product and caffeic acid | | | | | |
|---|---|---|---|---|---|
| | Group | DSS | Material | Dose | n |
| 1 | Normal group | No | distilled water | - | 3 |
| 2 | Control group | Yes | distilled water | - | 3 |
| 3 | NaNO₂ | Yes | sodium nitrite | 1 mM | 3 |
| 4 | NaNO₂+caffeic acid | Yes | sodium nitrite + caffeic acid | 1 mM | 3 |
| 5 | Fermented lettuce liquid | Yes | *Lactuca sativa* fermented extract | 1 mM | 3 |
| 6 | Fermented lettuce liquid+caffeic acid | Yes | *Lactuca sativa* fermented extract+ caffeic acid | 1 mM | 3 |
| 7 | Fermented soybean liquid | Yes | bean fermented extract | 1 mM | 3 |
| 8 | Fermented soybean liquid+caffeic acid | Yes | bean fermented extract + caffeic acid | 1 mM | 3 |
| 9 | Fermented garlic liquid | Yes | Garlic fermented extract | 1 mM | 3 |
| 10 | Fermented garlic liquid+caffeic acid | Yes | Garlic fermented extract + caffeic acid | 1 mM | 3 |
| 11 | Fermented bean sprout liquid | Yes | bean sprout fermented extract | 1 mM | 3 |
| 12 | Fermented bean sprout liquid+caffeic acid | Yes | bean sprout fermented extract + caffeic acid | 1 mM | 3 |
| 13 | Fermented cabbage liquid | Yes | Cabbage fermented extract | 1 mM | 3 |
| 14 | Fermented cabbage liquid+caffeic acid | Yes | Cabbage fermented extract +caffeic acid | 1 mM | 3 |
| 15 | Fermented whey liquid | Yes | Whey fermented extract | 1 mM | 3 |
| 16 | Fermented whey liquid+caffeic acid | Yes | Whey fermented extract + caffeic acid | 1 mM | 3 |

### 1-2. Measurement of disease activity index

Intestinal diseases were assessed based on clinical signs such as weight loss or diarrhea accompanied by bleeding and mucus, resulting in shortening of the colon. According to previous studies, the Disease Activity Index (DAI) comprises three primary clinical parameters: weight loss, diarrhea, and rectal bleeding. Weight loss is calculated as the difference between initial and current body weight. Diarrhea is defined as the presence of continuous soft fecal pellets in the rectum without the formation of pellet-like feces. The occurrence of rectal bleeding is considered separately from diarrhea with bleeding, total rectal bleeding, or other types of diarrhea. In the present invention, the DAI was calculated as follows:$DAI = \left(weight loss score\right) + \left(diarrhea score\right) + \left(rectal bleeding score\right)$

The medical parameters used herein represent a comprehensive functional assessment similar to the clinical symptoms observed in humans with ulcerative colitis (Table 3).

**[Table 3]**

| Disease activity index | | | |
|---|---|---|---|
| Score | Weight loss (%) | Diarrhea | Rectal bleeding |
| 0 | None | None | Normal |
| 1 | 1-5 | Mild | Occult |
| 2 | 6-10 | | |
| 3 | 11-15 | | |
| 4 | 16-20 | | |
| 5 | >20 | Severe watery | Gross bleeding |

### 1-3. Measurement of intestinal length and serum cytokines

At the end of the experiment, all experimental animals were subjected to orbital vein blood collection under inhalation anesthesia. Serum was separated, and a laparotomy was performed to excise the colon from the cecum to the distal end for measurement of intestinal length. Serum cytokine levels were measured using ELISA kits (R&D Systems, USA) to determine TNF-α and IL-1β production. Fifty microliters (50 µL) of assay diluent solution (ADS) were added to each antibody-coated well, followed by the addition of 50 µL of sample, and incubated at room temperature for 2 hours. Wells were then washed four times with 300 µL of wash buffer. After washing, 100 µL of mouse TNF-α or IL-1β as antibody was added to each well and incubated at room temperature for 2 hours, followed by removal and four washes. One hundred microliters (100 µL) of substrate solution were added to each well and incubated at room temperature for 30 minutes, after which 100 µL of stop solution was added. Absorbance was measured at 450 nm using an ELISA reader (SPECTRA max M2, Molecular Devices, USA).

### 1-4. Effect of the mixture in ulcerative colitis model

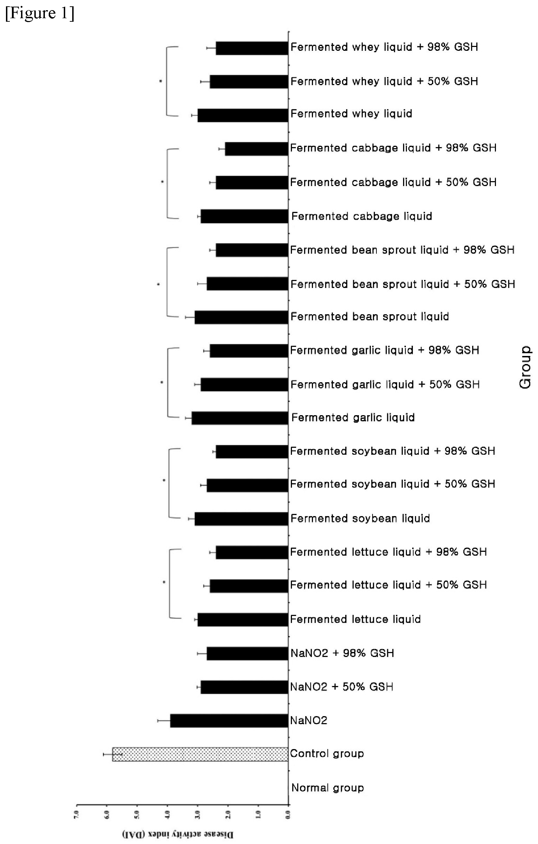

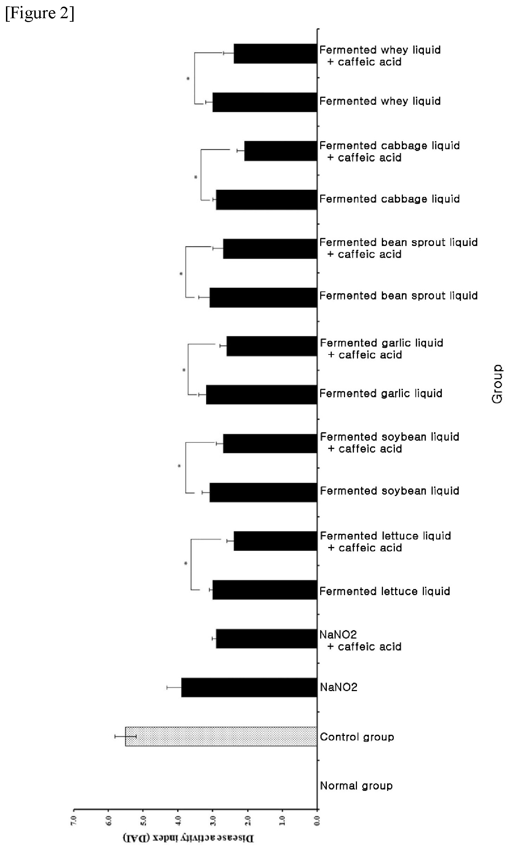

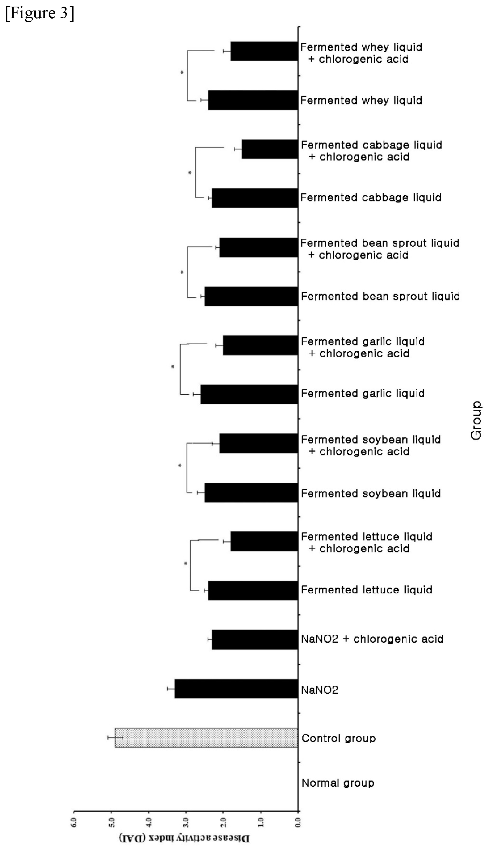

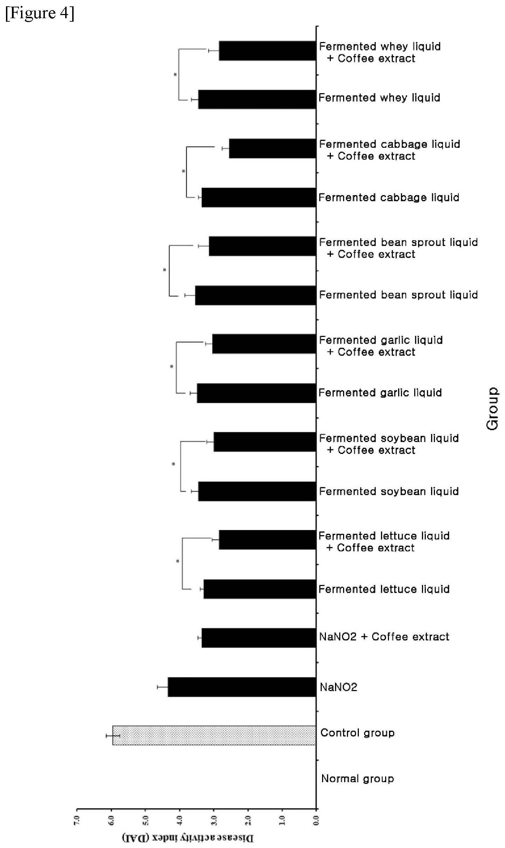

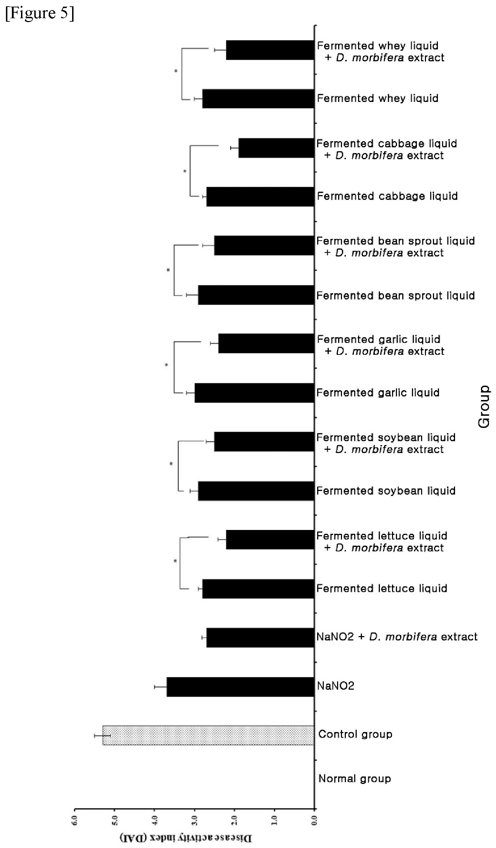

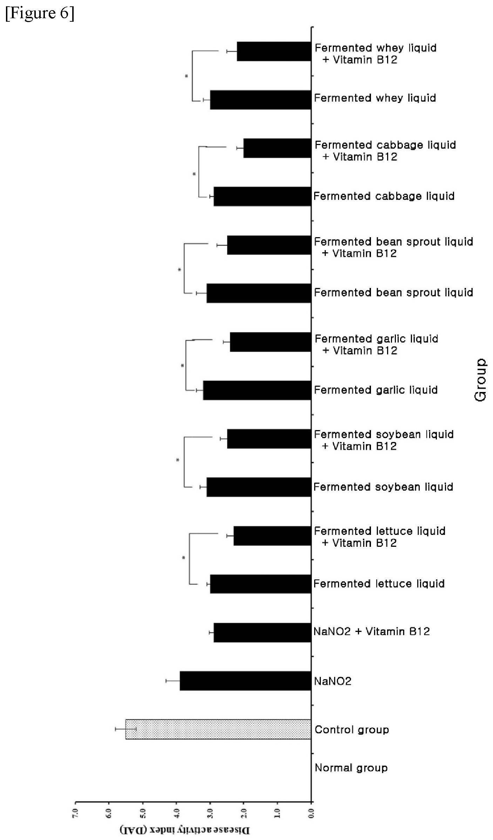

The DSS-induced colitis model exhibits clinical symptoms characterized by weight loss, diarrhea, and bloody fecal pellets. The effects of the test substances on these clinical symptoms were evaluated using the Disease Activity Index (DAI), and the results are shown in Figures 1 to 6. No symptoms were observed in the normal group, whereas the groups administered the test substances showed an overall reduction in DAI compared to the DSS control group. In particular, the groups receiving mixtures of glutathione, caffeic acid, chlorogenic acid, coffee extract, *Dendropanax morbifera* extract, or vitamin B12 in combination with the fermented liquid of natural product demonstrated a greater reduction in DAI compared to the group receiving only the fermented liquid of natural product.

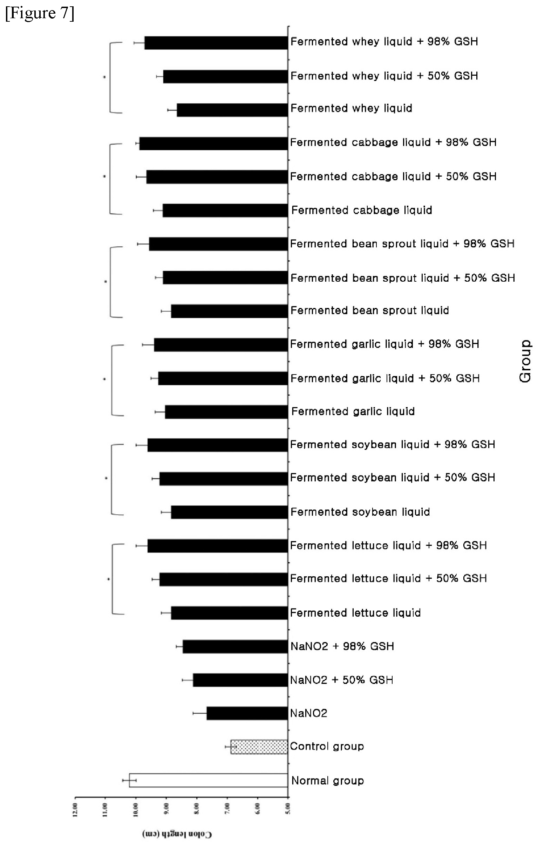

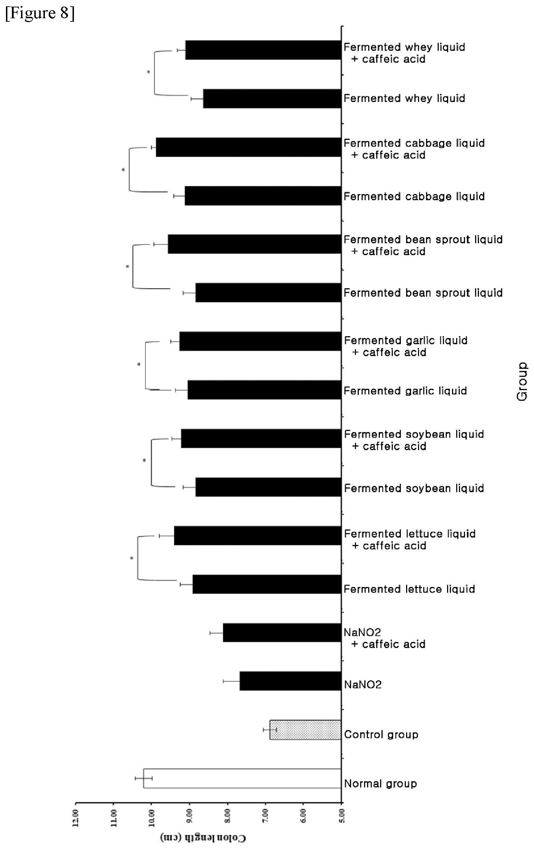

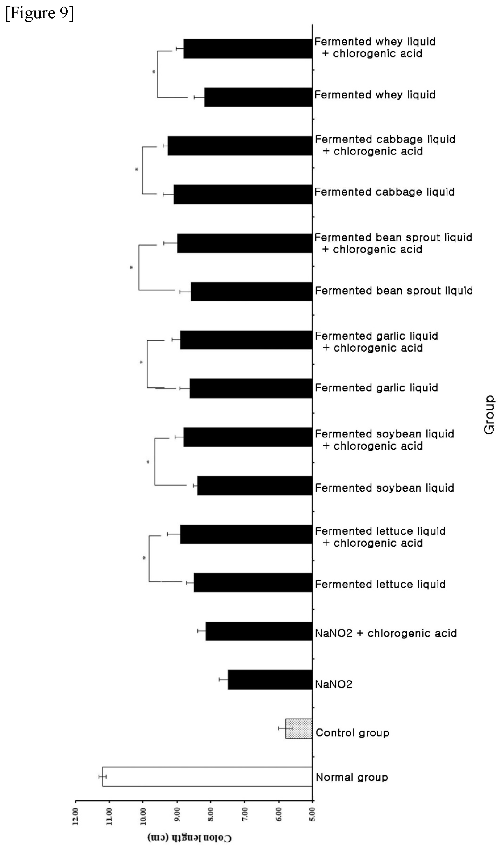

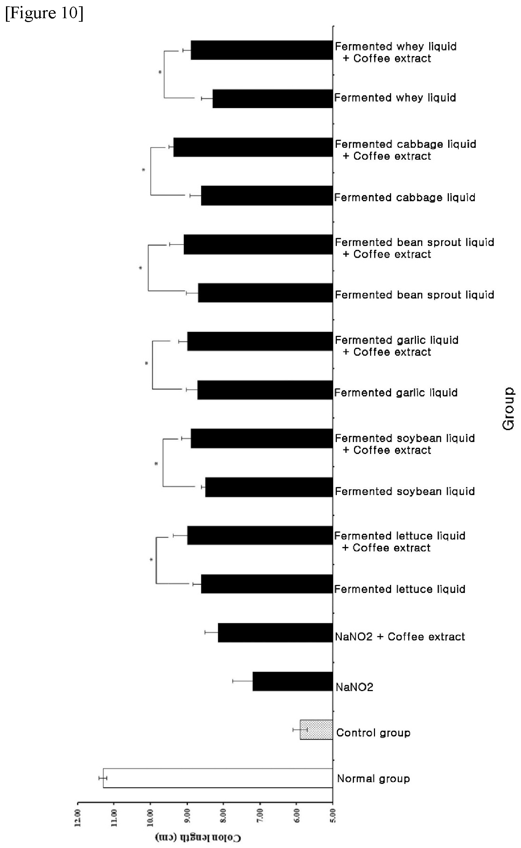

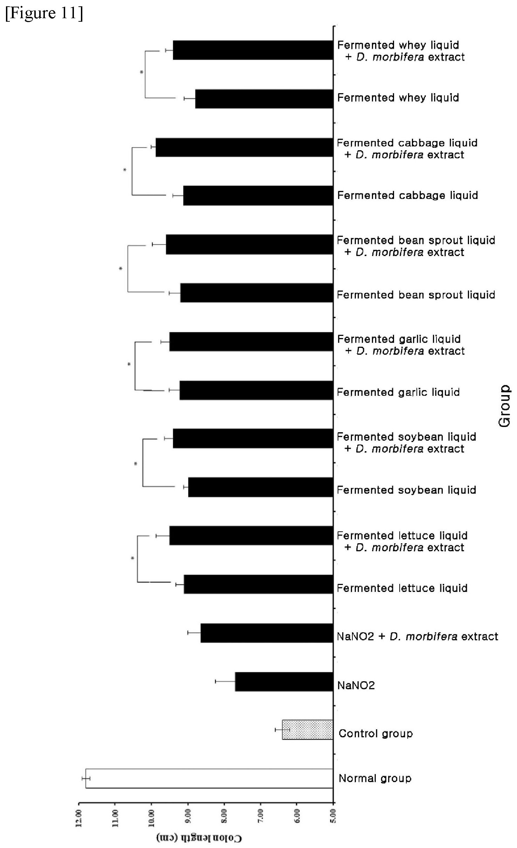

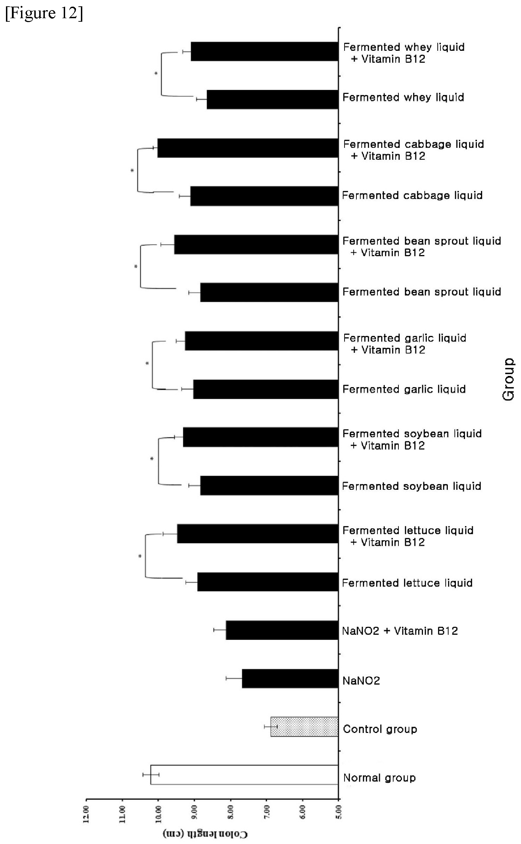

Furthermore, the colons of mice sacrificed on the fifth day after DSS administration were excised and their lengths measured. In all groups administered the test substances, colon length was greater than that of the control group. Compared to the NaNO₂-administered group, the group receiving the fermented liquid of natural product showed a longer colon. Moreover, in the groups receiving mixtures of glutathione, caffeic acid, chlorogenic acid, coffee extract, *Dendropanax morbifera* extract, or vitamin B12 in combination with the fermented liquid of natural product, colon length was closer to that of the normal group compared to the group receiving only the fermented liquid of natural product (Figures 7 to 12).

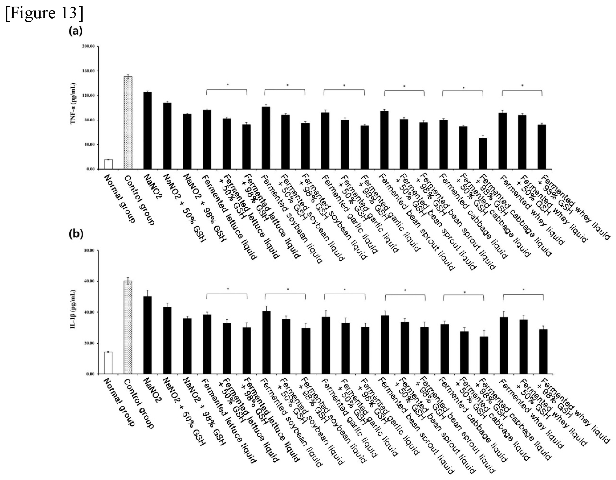

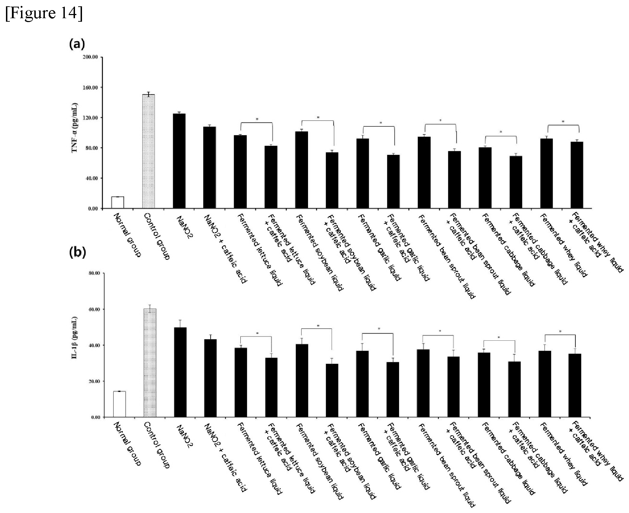

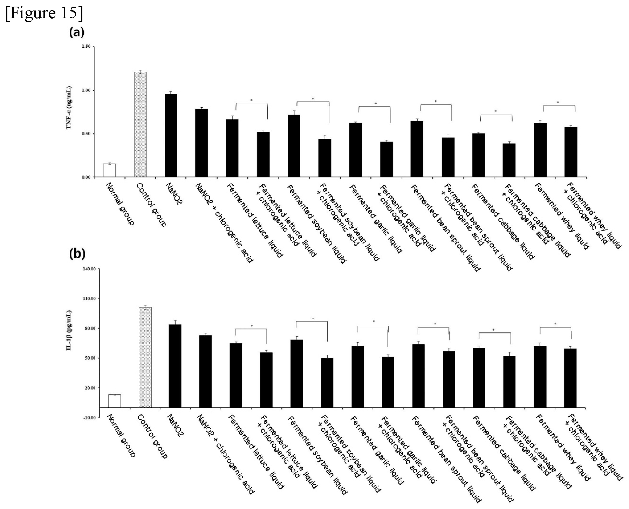

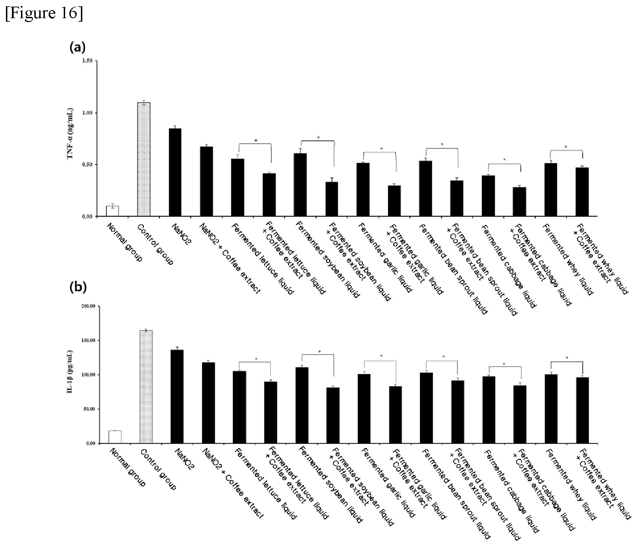

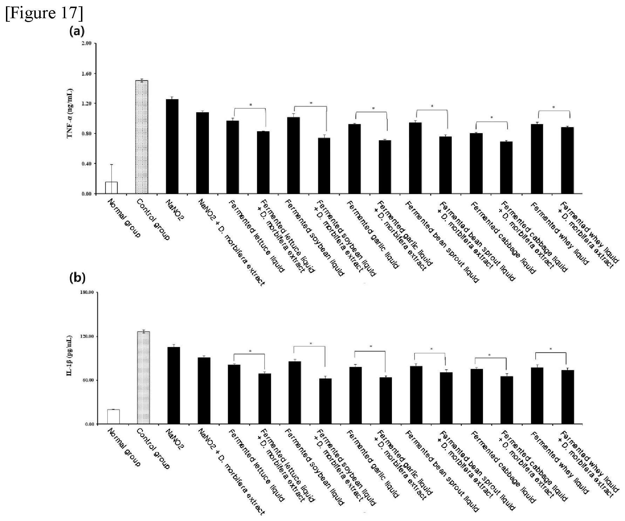

Furthermore, in the colitis-induced animal model, measurement of inflammatory cytokines in the blood showed that the levels of TNF-α and IL-1β, indicators of inflammation, were reduced in all groups administered the test substances compared to the control group. In particular, the lowest levels were observed in the groups receiving the mixture of glutathione, caffeic acid, chlorogenic acid, coffee extract, *Dendropanax morbifera* extract, or vitamin B12 in combination with the fermented liquid of natural product, compared to the group receiving only the fermented liquid of natural product (Figures 13 to 18).

### Example 2. Effect of mixture sample of fermented liquid containing nitric oxide metabolites and glutathione, caffeic acid, chlorogenic acid, coffee extract, Dendropanax morbifera extract, or vitamin B12 on amelioration of stress-induced irritable bowel syndrome

In the present experiment, eight-week-old female Wistar rats were purchased from Samtaco and acclimated for one week prior to use. The average body weight of the Wistar rats was 180.5±0.5 g, and the test substances were administered orally once daily. The animal housing room was maintained under a 12-hour light/dark cycle, with temperature controlled at 23±2°C and humidity maintained at 50-60%.

### 2-1. Group configuration and water avoidance stress (WAS)

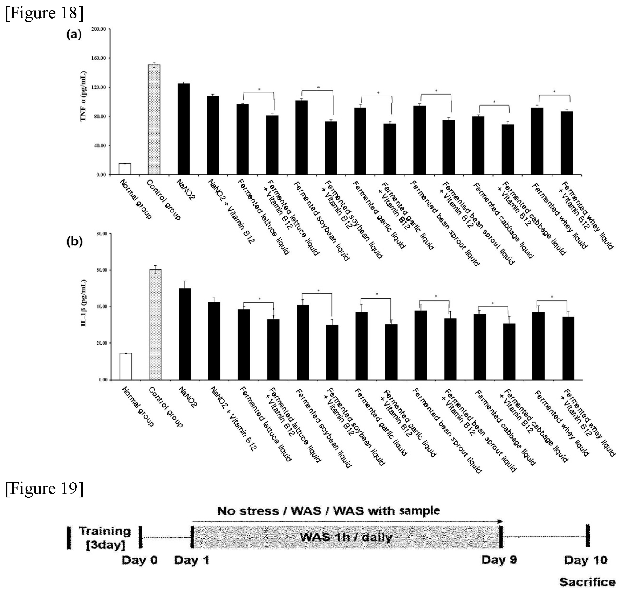

To observe changes in colonic motility induced by the mixture according to the present invention, all groups except the normal group were subjected to intermittent stress by placing the animals on a platform above a water-filled WAS tank, after which the number of fecal pellets was measured. During the administration of each test substance, daily water and feed intake were also recorded. WAS conducted for 60 minutes per day over a total of 8 days, divided into two sessions of 15 animals per hour to allow for intermediate replacement (Figure 19), during which fecal pellet counts were measured.

The group configuration was established as shown in the following Tables 4 and 5. The experimental groups receiving mixtures of chlorogenic acid, vitamin B12, coffee extract, and *Dendropanax morbifera* extract were configured identically to the caffeic acid mixture group shown in the following Table 5. Exposure to WAS induced diarrhea-like irritable bowel syndrome symptoms (increased defecation) and enhanced mucosal immunity, and the effects of administering each test substance on reducing these responses were observed.

**[Table 4]**

| Configuration of stress-induced irritable bowel syndrome experimental groups using mixture of fermented liquid of natural product and glutathione (GSH) | | | | | |
|---|---|---|---|---|---|
| | Group | WAS | Material | Dose | n |
| 1 | Normal group | No | distilled water | - | 5 |
| 2 | Control group | Yes | distilled water | - | 5 |
| 3 | NaNO₂ | Yes | sodium nitrite | 1 mM | 5 |
| 4 | NaNO₂+50% GSH | Yes | sodium nitrite + Yeast extract 50% glutathione | 1 mM | 5 |
| 5 | NaNO₂+98% GSH | Yes | sodium nitrite + 98% glutathione | 1 mM | 5 |
| 6 | Fermented lettuce liquid | Yes | *Lactuca sativa* fermented extract | 1 mM | 5 |
| 7 | Fermented lettuce liquid+50% GSH | Yes | *Lactuca sativa* fermented extract + Yeast extract 50% glutathione | 1 mM | 5 |
| 8 | Fermented lettuce liquid+98% GSH | Yes | *Lactuca sativa* fermented extract + 98% glutathione | 1 mM | 5 |
| 9 | Fermented soybean liquid | Yes | bean fermented extract | 1 mM | 5 |
| 10 | Fermented soybean liquid+50% GSH | Yes | bean fermented extract + Yeast extract 50% glutathione | 1 mM | 5 |
| 11 | Fermented soybean liquid+98% GSH | Yes | bean fermented extract + 98% glutathione | 1 mM | 5 |
| 12 | Fermented garlic liquid | Yes | Garlic fermented extract | 1 mM | 5 |
| 13 | Fermented garlic liquid+50% GSH | Yes | Garlic fermented extract + Yeast extract 50% glutathione | 1 mM | 5 |
| 14 | Fermented garlic liquid+98% GSH | Yes | Garlic fermented extract + 98% glutathione | 1 mM | 5 |
| 15 | Fermented bean sprout liquid | Yes | bean sprout fermented extract | 1 mM | 5 |
| 16 | Fermented bean sprout liquid+50% GSH | Yes | bean sprout fermented extract + Yeast extract 50% glutathione | 1 mM | 5 |
| 17 | Fermented bean sprout liquid+98% GSH | Yes | bean sprout fermented extract + 98% glutathione | 1 mM | 5 |
| 18 | Fermented cabbage liquid | Yes | Cabbage fermented extract | 1 mM | 5 |
| 19 | Fermented cabbage liquid+50% GSH | Yes | Cabbage fermented extract + Yeast extract 50% glutathione | 1 mM | 5 |
| 20 | Fermented cabbage liquid+98% GSH | Yes | Cabbage fermented extract + 98% glutathione | 1 mM | 5 |
| 21 | Fermented whey liquid | Yes | Whey fermented extract | 1 mM | 5 |
| 22 | Fermented whey liquid+50% GSH | Yes | Whey fermented extract + Yeast extract 50% glutathione | 1 mM | 5 |
| 23 | Fermented whey liquid+98% GSH | Yes | Whey fermented extract + 98% glutathione | 1 mM | 5 |

**[Table 5]**

| Configuration of stress-induced irritable bowel syndrome experimental groups using mixture of fermented liquid of natural product and caffeic acid | | | | | |
|---|---|---|---|---|---|
| | Group | WAS | Material | Dose | n |
| 1 | Normal group | No | distilled water | - | 3 |
| 2 | Control group | Yes | distilled water | - | 3 |
| 3 | NaNO₂ | Yes | sodium nitrite | 1 mM | 3 |
| 4 | NaNO₂+caffeic acid | Yes | sodium nitrite + caffeic acid | 1 mM | 3 |
| 5 | Fermented lettuce liquid | Yes | *Lactuca sativa* fermented extract | 1 mM | 3 |
| 6 | Fermented lettuce liquid+caffeic acid | Yes | *Lactuca sativa* fermented extract+ caffeic acid | 1 mM | 3 |
| 7 | Fermented soybean liquid | Yes | bean fermented extract | 1 mM | 3 |
| 8 | Fermented soybean liquid+caffeic acid | Yes | bean fermented extract + caffeic acid | 1 mM | 3 |
| 9 | Fermented garlic liquid | Yes | Garlic fermented extract | 1 mM | 3 |
| 10 | Fermented garlic liquid+caffeic acid | Yes | Garlic fermented extract + caffeic acid | 1 mM | 3 |
| 11 | Fermented bean sprout liquid | Yes | bean sprout fermented extract | 1 mM | 3 |
| 12 | Fermented bean sprout liquid+caffeic acid | Yes | bean sprout fermented extract + caffeic acid | 1 mM | 3 |
| 13 | Fermented cabbage liquid | Yes | Cabbage fermented extract | 1 mM | 3 |
| 14 | Fermented cabbage liquid+caffeic acid | Yes | Cabbage fermented extract +caffeic acid | 1 mM | 3 |
| 15 | Fermented whey liquid | Yes | Whey fermented extract | 1 mM | 3 |
| 16 | Fermented whey liquid+caffeic acid | Yes | Whey fermented extract + caffeic acid | 1 mM | 3 |

### 2-2. Effect of mixture in stress-induced irritable bowel syndrome model

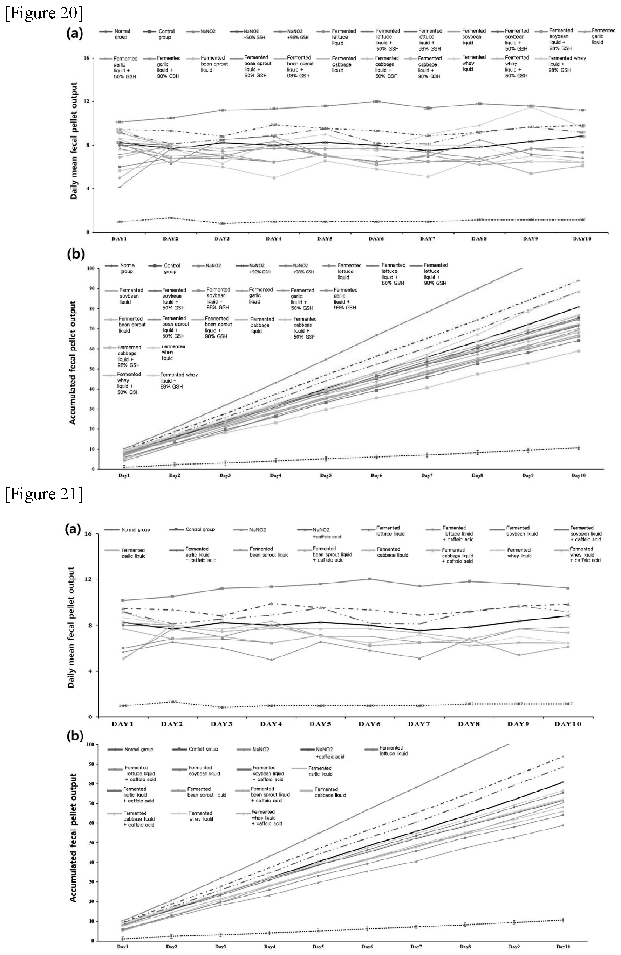

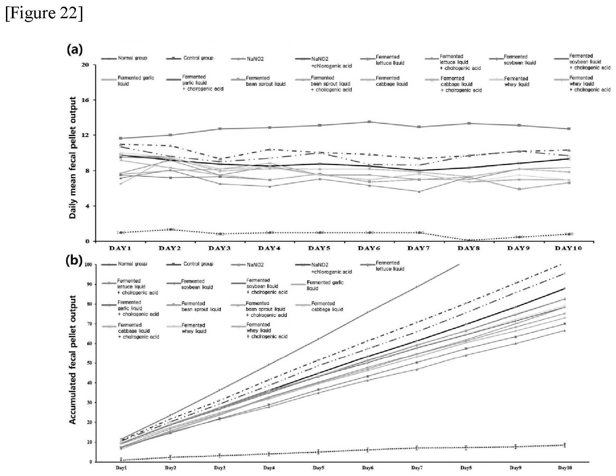

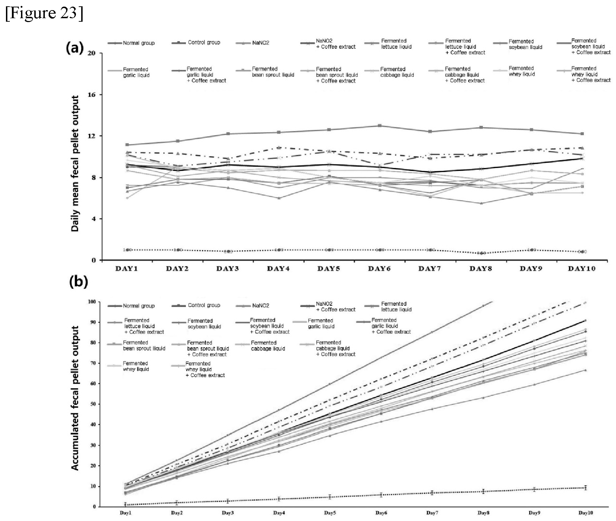

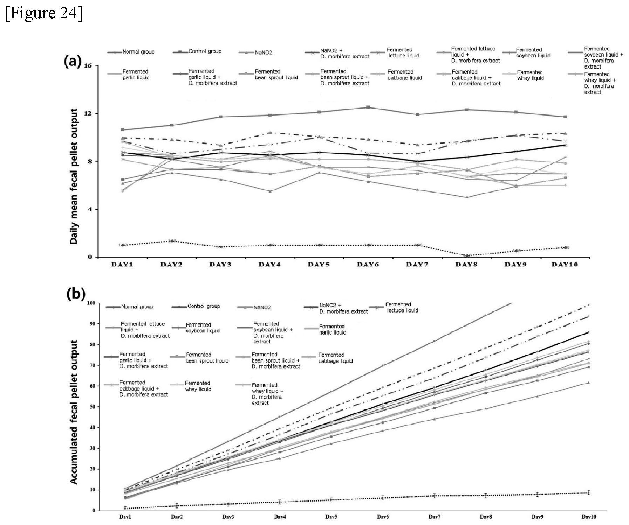

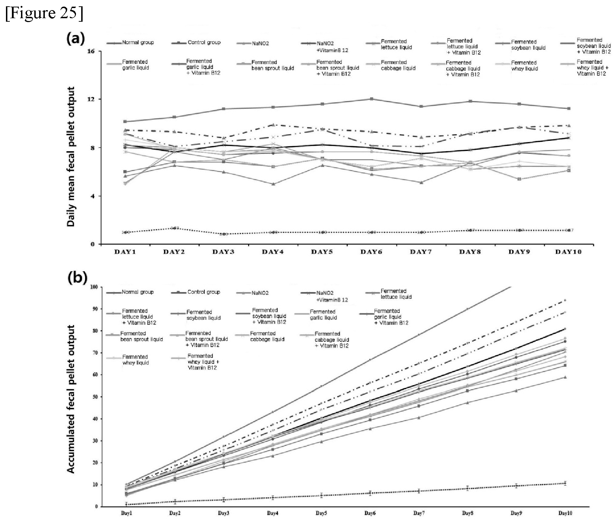

Measurement of fecal pellet counts following stress induction via WAS is shown in Figures 20 to 25. In all groups administered the test substances, fecal pellet counts were reduced compared to the control group. In particular, the groups receiving mixtures of glutathione, caffeic acid, chlorogenic acid, coffee extract, *Dendropanax morbifera* extract, or vitamin B12 in combination with the fermented liquid of natural product showed a further reduction in fecal pellet counts compared to the group receiving only the fermented liquid of natural product.

### Example 3. Effect of mixture sample of fermented liquid containing nitric oxide metabolites and glutathione, caffeic acid, chlorogenic acid, coffee extract, Dendropanax morbifera extract, or vitamin B12 on amelioration of blood pressure

In this experiment, seven-week-old male Sprague Dawley rats (hereinafter, SD rats) were purchased from Samtaco and acclimated for one week prior to use. The average body weight of the SD rats was 198.95±2.10 g, and the test substances were administered orally before the experiment. The animal housing room was maintained under a 12-hour light/dark cycle, with temperature controlled at 23±2°C and humidity maintained at 50-60%.

### 3-1. DOCA-salt hypertension rat model preparation and group configuration

The hypertension model was established by intraperitoneally administering DOCA (deoxycorticosterone acetate; TCI, Japan) at a dose of 25 mg/kg twice weekly for four weeks to eight-week-old male SD rats, while providing 1% sodium chloride solution as drinking water to induce hypertension. The control group received distilled water (DW), and the experimental groups were administered the respective test substances. To determine the induction of hypertension, blood pressure was measured using a mouse tail blood pressure system (MK-2000STS, MUROMACHI KIKAI, Japan), and animals with systolic blood pressure of 130 mmHg or higher were selected for use. Blood pressure measurements were conducted in real time for 55 minutes per animal after administration of each test substance. Rats were secured in a restraining frame, and a piezoelectric pulse sensor and occlusion cuff were applied to the tail, connected to a computer to observe pulse signals. The device was then operated to monitor systolic blood pressure. The experimental groups were configured as shown in the following Tables 6 and 7. The experimental groups receiving mixtures of chlorogenic acid, vitamin B12, coffee extract, and *Dendropanax morbifera* extract were configured identically to the caffeic acid mixture group shown in the following Table 7.

**[Table 6]**

| Configuration of blood pressure experimental groups using mixture of fermented liquid of natural product and glutathione (GSH) | | | | | | |
|---|---|---|---|---|---|---|
| Group | Treatment | | | | n | |
| | DOCA | Dose | material | Dose | | |
| | | | | | | |
| 1 | Normal group | No | 25 mg/kg | distilled water | - | 5 |
| 2 | Control group | Yes | 25 mg/kg | distilled water | - | 5 |
| 3 | NaNO₂ | Yes | 25 mg/kg | sodium nitrite | 1 mM | 5 |
| 4 | NaNO₂+50 % GSH | Yes | 25 mg/kg | sodium nitrite + Yeast extract 50% glutathione | 1 mM | 5 |
| 5 | NaNO₂+98 % GSH | Yes | 25 mg/kg | sodium nitrite + 98% glutathione | 1 mM | 5 |
| 6 | Fermented lettuce liquid | Yes | 25 mg/kg | *Lactuca sativa* fermented extract | 1 mM | 5 |
| 7 | Fermented lettuce liquid+50% GSH | Yes | 25 mg/kg | *Lactuca sativa* fermented extract + Yeast extract 50% glutathione | 1 mM | 5 |
| 8 | Fermented lettuce liquid+98% GSH | Yes | 25 mg/kg | *Lactuca sativa* fermented extract + 98% glutathione | 1 mM | 5 |
| 9 | Fermented soybean liquid | Yes | 25 mg/kg | bean fermented extract | 1 mM | 5 |
| 10 | Fermented soybean liquid+50% GSH | Yes | 25 mg/kg | bean fermented extract + Yeast extract 50% glutathione | 1 mM | 5 |
| 11 | Fermented soybean liquid+98% GSH | Yes | 25 mg/kg | bean fermented extract + 98% glutathione | 1 mM | 5 |
| 12 | Fermented garlic liquid | Yes | 25 mg/kg | Garlic fermented extract | 1 mM | 5 |
| 13 | Fermented garlic liquid+50% GSH | Yes | 25 mg/kg | Garlic fermented extract + Yeast extract 50% glutathione | 1 mM | 5 |
| 14 | Fermented garlic liquid+98% GSH | Yes | 25 mg/kg | Garlic fermented extract + 98% glutathione | 1 mM | 5 |
| 15 | Fermented bean sprout liquid | Yes | 25 mg/kg | bean sprout fermented extract | 1 mM | 5 |
| 16 | Fermented bean sprout liquid+50% GSH | Yes | 25 mg/kg | bean sprout fermented extract + Yeast extract 50% glutathione | 1 mM | 5 |
| 17 | Fermented bean sprout liquid+98% GSH | Yes | 25 mg/kg | bean sprout fermented extract + 98% glutathione | 1 mM | 5 |
| 18 | Fermented cabbage liquid | Yes | 25 mg/kg | Cabbage fermented extract | 1 mM | 5 |
| 19 | Fermented cabbage liquid+50% GSH | Yes | 25 mg/kg | Cabbage fermented extract + Yeast extract 50% glutathione | 1 mM | 5 |
| 20 | Fermented cabbage liquid+98% GSH | Yes | 25 mg/kg | Cabbage fermented extract + 98% glutathione | 1 mM | 5 |
| 21 | Fermented whey liquid | Yes | 25 mg/kg | Whey fermented extract | 1 mM | 5 |
| 22 | Fermented whey liquid+50% GSH | Yes | 25 mg/kg | Whey fermented extract + Yeast extract 50% glutathione | 1 mM | 5 |
| 23 | Fermented whey liquid+98% GSH | Yes | 25 mg/kg | Whey fermented extract + 98% glutathione | 1 mM | 5 |

**[Table 7]**

| Configuration of blood pressure experimental groups using mixture of fermented liquid of natural product and caffeic acid | | | | | | |
|---|---|---|---|---|---|---|
| Group | | Treatment | | | | n |
| | | DOCA | Dose | material | Dose | |
| 1 | Normal group | No | 25 mg/kg | distilled water | - | 3 |
| 2 | Control group | Yes | 25 mg/kg | distilled water | - | 3 |
| 3 | NaNO₂ | Yes | 25 mg/kg | sodium nitrite | 1 mM | 3 |
| 4 | NaNO₂+caf feic acid | Yes | 25 mg/kg | sodium nitrite + caffeic acid | 1 mM | 3 |
| 5 | Fermented lettuce liquid | Yes | 25 mg/kg | *Lactuca sativa* fermented extract | 1 mM | 3 |
| 6 | Fermented lettuce liquid+caffeic acid | Yes | 25 mg/kg | *Lactuca sativa* fermented extract+ caffeic acid | 1 mM | 3 |
| 7 | Fermented soybean liquid | Yes | 25 mg/kg | bean fermented extract | 1 mM | 3 |
| 8 | Fermented soybean liquid+caff eic acid | Yes | 25 mg/kg | bean fermented extract + caffeic acid | 1 mM | 3 |
| 9 | Fermented garlic liquid | Yes | 25 mg/kg | Garlic fermented extract | 1 mM | 3 |
| 10 | Fermented garlic liquid+caff eic acid | Yes | 25 mg/kg | Garlic fermented extract + caffeic acid | 1 mM | 3 |
| 11 | Fermented bean sprout liquid | Yes | 25 mg/kg | bean sprout fermented extract | 1 mM | 3 |
| 12 | Fermented bean sprout liquid+caff eic acid | Yes | 25 mg/kg | bean sprout fermented extract + caffeic acid | 1 mM | 3 |
| 13 | Fermented cabbage liquid | Yes | 25 mg/kg | Cabbage fermented extract | 1 mM | 3 |
| 14 | Fermented cabbage liquid+caff eic acid | Yes | 25 mg/kg | Cabbage fermented extract +caffeic acid | 1 mM | 3 |
| 15 | Fermented whey liquid | Yes | 25 mg/kg | Whey fermented extract | 1 mM | 3 |
| 16 | Fermented whey liquid+caff eic acid | Yes | 25 mg/kg | Whey fermented extract + caffeic acid | 1 mM | 3 |

### 3-2. Effect of mixture in DOCA-induced hypertension model

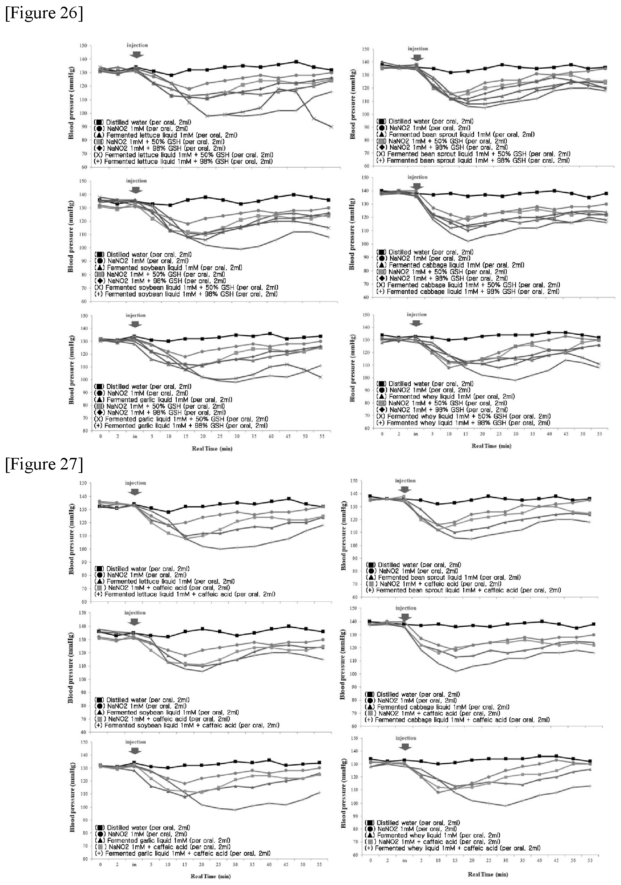

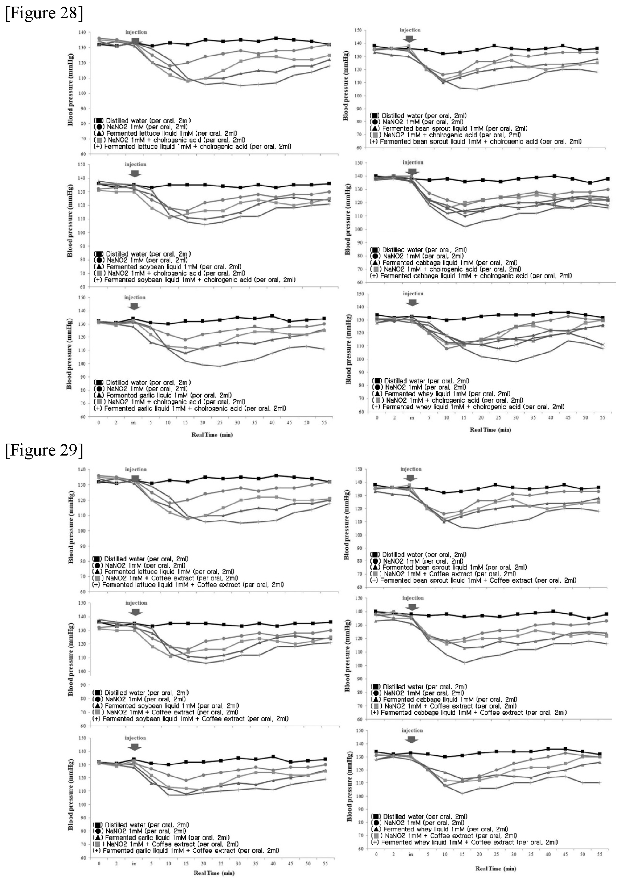

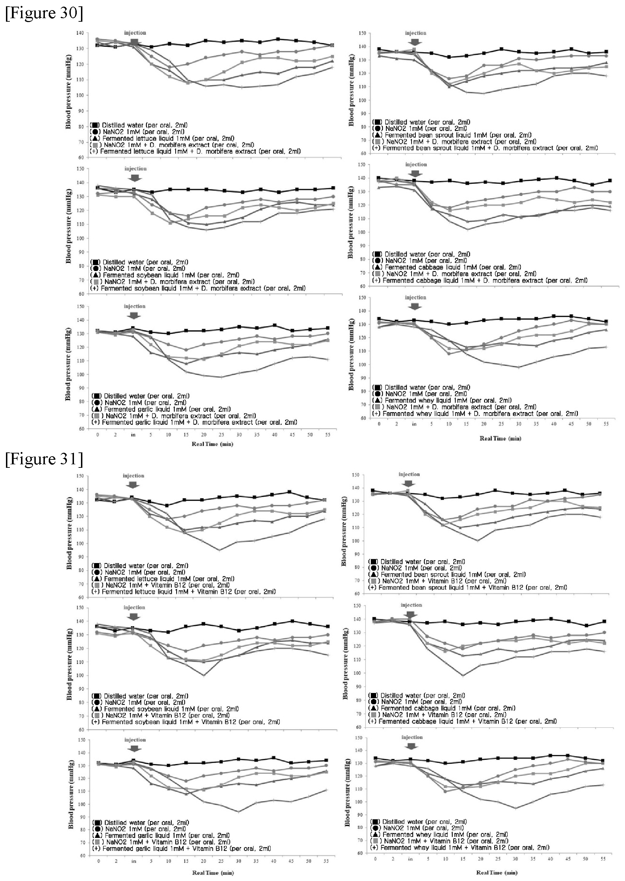

Measurement of blood pressure in rats with hypertension induced by DOCA-salt before and after administration of each fermented liquid is shown in Figures 26 to 31. A reduction in blood pressure was observed between 10 and 20 minutes after oral administration of the test substances, with the greatest decrease observed in the groups receiving a mixture of the fermented liquid of natural product with glutathione, caffeic acid, chlorogenic acid, coffee extract, *Dendropanax morbifera* extract, or vitamin B12.

### Example 4. Effect of mixture sample of fermented liquid containing nitric oxide metabolites and glutathione, caffeic acid, chlorogenic acid, coffee extract, Dendropanax morbifera extract, or vitamin B12 on amelioration of arthritis

In this experiment, seven-week-old male SD rats were purchased from Samtaco and acclimated for one week prior to use. The test substances were administered orally once daily for four weeks. The animal housing room was maintained under a 12-hour light/dark cycle, with temperature controlled at 23±2°C and humidity maintained at 50-60%.

### 4-1. Induction of arthritis and group configuration

Arthritis was induced in all experimental groups except the normal group by first anesthetizing the animals and shaving the area around the left knee. Then, monosodium iodoacetate (MIA), a substance for inducing osteoarthritis, was administered intra-articularly into the left knee joint at a volume of 50 µL (60-80 mg/mL) using a 31G insulin syringe (0.25×8, BD Medical-Diabetes Care, USA). MIA was diluted with 0.9% saline, and seven days after MIA injection, the respective test substances were administered to each experimental group. The experimental groups were configured as shown in the following Tables 8 and 9. The groups receiving mixtures of chlorogenic acid, vitamin B12, coffee extract, and *Dendropanax morbifera* extract were configured identically to the caffeic acid mixture group shown in the following Table 9.

**[Table 8]**

| Configuration of arthritis experimental groups using mixture of fermented liquid of natural product and glutathione (GSH) | | | | | |
|---|---|---|---|---|---|
| Group | | MIA | Material | Dose | n |
| 1 | Normal group | No | distilled water | - | 5 |
| 2 | Control group | Yes | distilled water | - | 5 |
| 3 | NaNO₂ | Yes | sodium nitrite | 1 mM | 5 |
| 4 | NaNO₂+50% GSH | Yes | sodium nitrite + Yeast extract 50% glutathione | 1 mM | 5 |
| 5 | NaNO₂+98% GSH | Yes | sodium nitrite + 98% glutathione | 1 mM | 5 |
| 6 | Fermented lettuce liquid | Yes | *Lactuca sativa* fermented extract | 1 mM | 5 |
| 7 | Fermented lettuce liquid+50% GSH | Yes | *Lactuca sativa* fermented extract + Yeast extract 50% glutathione | 1 mM | 5 |
| 8 | Fermented lettuce liquid+98% GSH | Yes | *Lactuca sativa* fermented extract + 98% glutathione | 1 mM | 5 |
| 9 | Fermented soybean liquid | Yes | bean fermented extract | 1 mM | 5 |
| 10 | Fermented soybean liquid+50% GSH | Yes | bean fermented extract + Yeast extract 50% glutathione | 1 mM | 5 |
| 11 | Fermented soybean liquid+98% GSH | Yes | bean fermented extract + 98% glutathione | 1 mM | 5 |
| 12 | Fermented garlic liquid | Yes | Garlic fermented extract | 1 mM | 5 |
| 13 | Fermented garlic liquid+50% GSH | Yes | Garlic fermented extract + Yeast extract 50% glutathione | 1 mM | 5 |
| 14 | Fermented garlic liquid+98% GSH | Yes | Garlic fermented extract + 98% glutathione | 1 mM | 5 |
| 15 | Fermented bean sprout liquid | Yes | bean sprout fermented extract | 1 mM | 5 |
| 16 | Fermented bean sprout liquid+50% GSH | Yes | bean sprout fermented extract + Yeast extract 50% glutathione | 1 mM | 5 |
| 17 | Fermented bean sprout liquid+98% GSH | Yes | bean sprout fermented extract + 98% glutathione | 1 mM | 5 |
| 18 | Fermented cabbage liquid | Yes | Cabbage fermented extract | 1 mM | 5 |
| 19 | Fermented cabbage liquid+50% GSH | Yes | Cabbage fermented extract + Yeast extract 50% glutathione | 1 mM | 5 |
| 20 | Fermented cabbage liquid+98% GSH | Yes | Cabbage fermented extract + 98% glutathione | 1 mM | 5 |
| 21 | Fermented whey liquid | Yes | Whey fermented extract | 1 mM | 5 |
| 22 | Fermented whey liquid+50% GSH | Yes | Whey fermented extract + Yeast extract 50% glutathione | 1 mM | 5 |
| 23 | Fermented whey liquid+98% GSH | Yes | Whey fermented extract + 98% glutathione | 1 mM | 5 |

**[Table 9]**

| Configuration of arthritis experimental groups using mixture of fermented liquid of natural product and caffeic acid | | | | | |
|---|---|---|---|---|---|
| Group | | MIA | Material | Dose | n |
| 1 | Normal group | No | distilled water | - | 3 |
| 2 | Control group | Yes | distilled water | - | 3 |
| 3 | NaNO₂ | Yes | sodium nitrite | 1 mM | 3 |
| 4 | NaNO₂+caffeic acid | Yes | sodium nitrite + caffeic acid | 1 mM | 3 |
| 5 | Fermented lettuce liquid | Yes | *Lactuca sativa* fermented extract | 1 mM | 3 |
| 6 | Fermented lettuce liquid+caffeic acid | Yes | *Lactuca sativa* fermented extract+ caffeic acid | 1 mM | 3 |
| 7 | Fermented soybean liquid | Yes | bean fermented extract | 1 mM | 3 |
| 8 | Fermented soybean liquid+caffeic acid | Yes | bean fermented extract + caffeic acid | 1 mM | 3 |
| 9 | Fermented garlic liquid | Yes | Garlic fermented extract | 1 mM | 3 |
| 10 | Fermented garlic liquid+caffeic acid | Yes | Garlic fermented extract + caffeic acid | 1 mM | 3 |
| 11 | Fermented bean sprout liquid | Yes | bean sprout fermented extract | 1 mM | 3 |
| 12 | Fermented bean sprout liquid+caffeic acid | Yes | bean sprout fermented extract + caffeic acid | 1 mM | 3 |
| 13 | Fermented cabbage liquid | Yes | Cabbage fermented extract | 1 mM | 3 |
| 14 | Fermented cabbage liquid+caffeic acid | Yes | Cabbage fermented extract +caffeic acid | 1 mM | 3 |
| 15 | Fermented whey liquid | Yes | Whey fermented extract | 1 mM | 3 |
| 16 | Fermented whey liquid+caffeic acid | Yes | Whey fermented extract + caffeic acid | 1 mM | 3 |

### 4-2. Measurement of leg edema thickness

After induction of arthritis with MIA, changes in leg edema thickness were measured at the left knee joint of the rats. Leg edema thickness was measured under anesthesia using Vernier calipers (Mitutoyo Co., Japan).

### 4-3. Measurement of arthritis index (severity score)

For the evaluation of the arthritis index, before sacrificing the model animals, each animal was photographed, and the arthritis index was visually evaluated by three examiners. The evaluation method was modified from Kim et al. (Korean J Orient Physiol Pathol, Effects of Acanthopanax senticosus and onion mixture extract on the collagen-induced arthritis in rat model, 2011, 25; 1000-1007). The three examiners independently observed each animal three times, assigning scores from 1 to 5, and the results were expressed as the average value (Table 10).

**[Table 10]**

| Clinical evaluation of arthritis | |
|---|---|
| Level | Clinical symptoms |
| 1 | No change |
| 2 | Mild erythema of the ankle joint |
| 3 | Mild swelling and erythema of the ankle joint |
| 4 | Moderate swelling and erythema of the ankle joint through the metatarsal bone |
| 5 | Severe swelling and erythema of the ankle joint through the digit |

### 4-4. Measurement of production of inflammatory cytokine and inflammatory mediator in serum

Serum cytokine levels were measured using ELISA kits (R&D Systems, USA) to determine TNF-α and metallopeptidase-9 (MMP-9) production. Fifty microliters (50 µL) of assay diluent solution (ADS) were added to each antibody-coated well, followed by 50 µL of sample, and incubated at room temperature for 2 hours. The wells were then washed four times with 300 µL of wash buffer. After washing, 100 µL of mouse TNF-α or MMP-9 as antibody was added to each well and incubated at room temperature for 2 hours, followed by removal and four washes. One hundred microliters (100 µL) of substrate solution were added to each well and incubated at room temperature for 30 minutes, after which 100 µL of stop solution was added. Absorbance was measured at 450 nm using an ELISA reader (SPECTRA max M2, Molecular Devices, USA).

### 4-5. Measurement of PGE₂ (Prostaglandin E₂) in serum

Serum PGE₂ levels were measured using an ELISA kit. Fifty microliters (50 µL) of assay diluent solution (ADS) were added to each antibody-coated well, followed by 50 µL of sample, and incubated at room temperature for 2 hours. The wells were then washed four times with 300 µL of wash buffer. After washing, 50 µL of proteoglycan E2 conjugate as antibody was added to each well and incubated at room temperature for 2 hours, followed by removal and four washes. Two hundred microliters (200 µL) of substrate solution were added to each well and incubated at room temperature for 30 minutes, after which 100 µL of stop solution was added. Absorbance was measured at 450 nm using an ELISA reader (Molecular Devices).

### 4-6. Effect of mixture in arthritis model

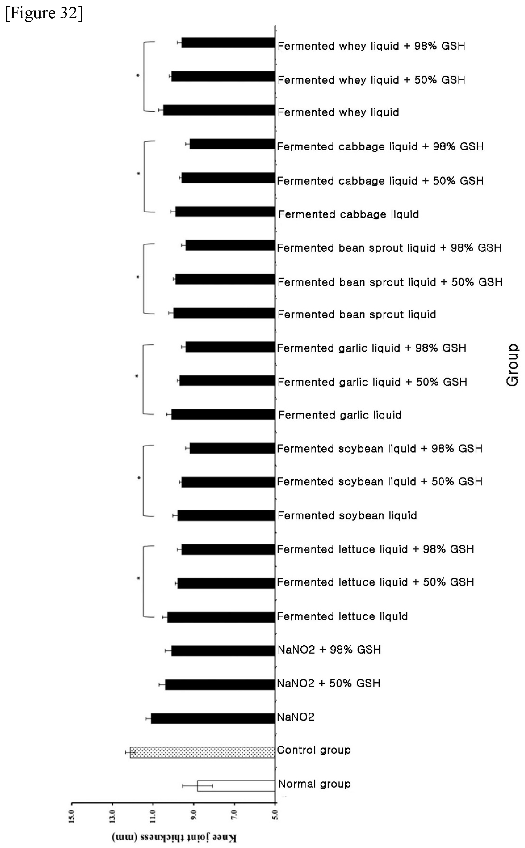

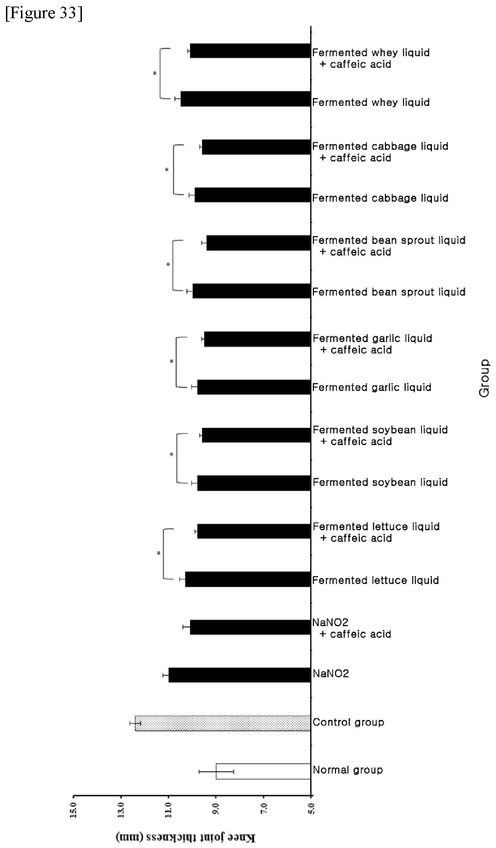

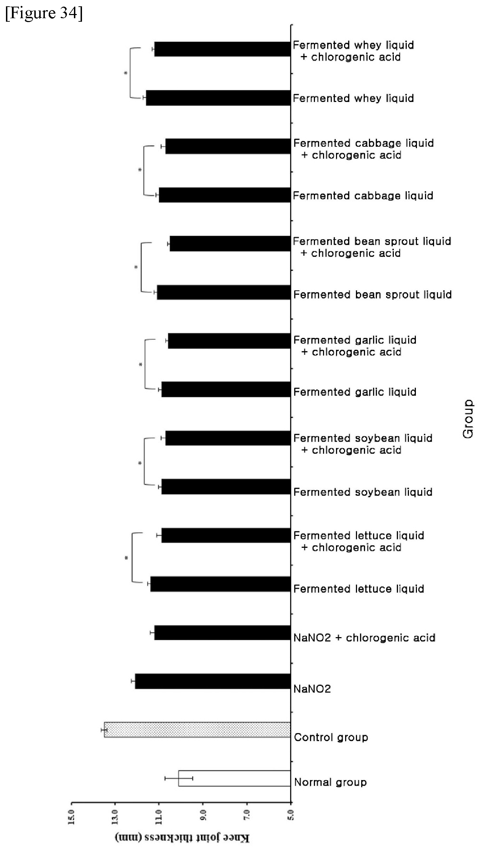

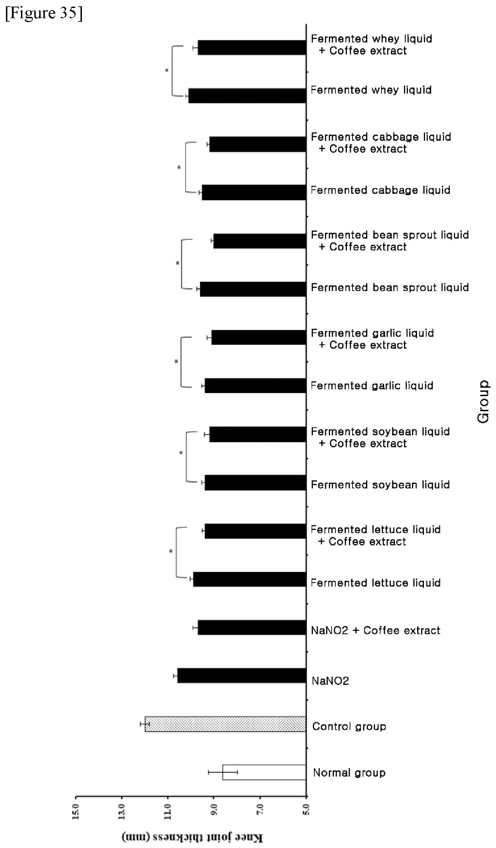

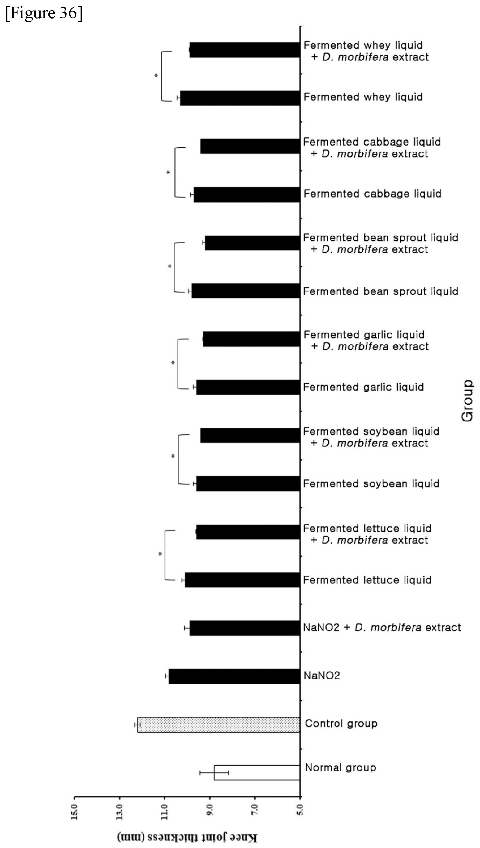

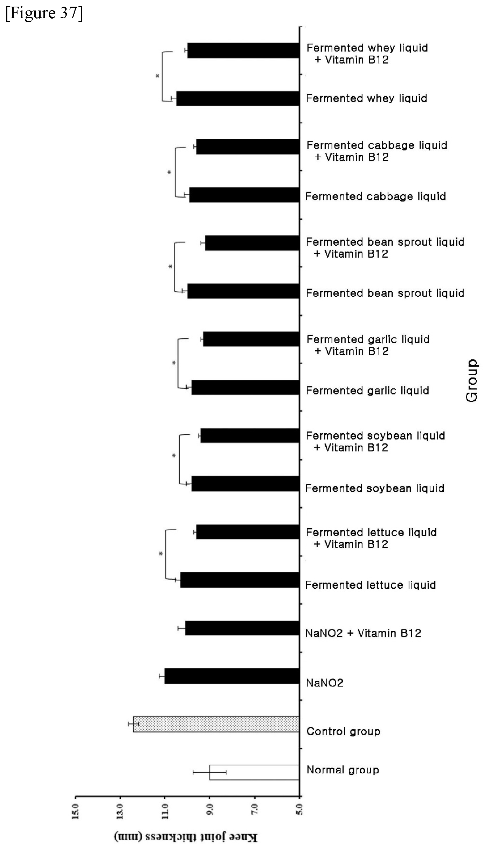

At the end of the experiment, the left knee joint of each experimental group was examined to assess morphological changes indicating the degree of arthritis alleviation. In the control group treated with MIA alone, an increase in leg thickness due to edema was observed compared to the untreated normal group, whereas administration of the test substances resulted in a reduction in the thickness (Figures 32 to 37).

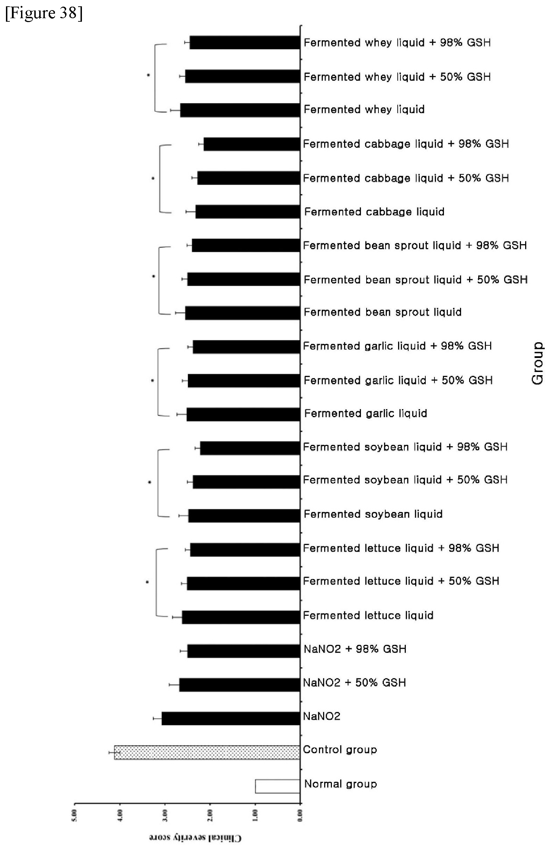

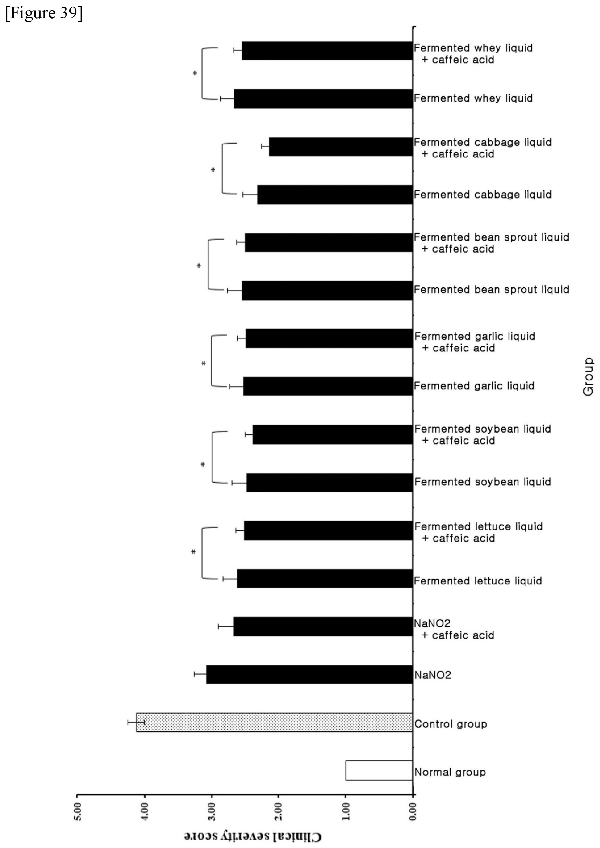

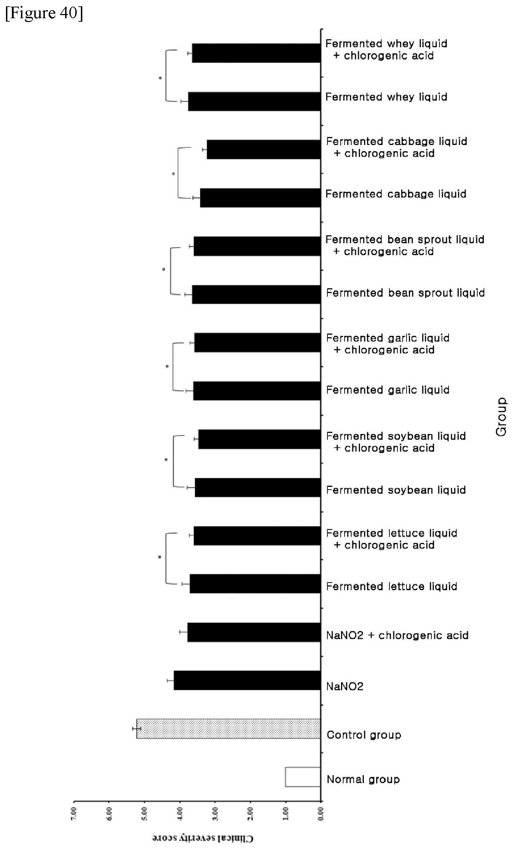

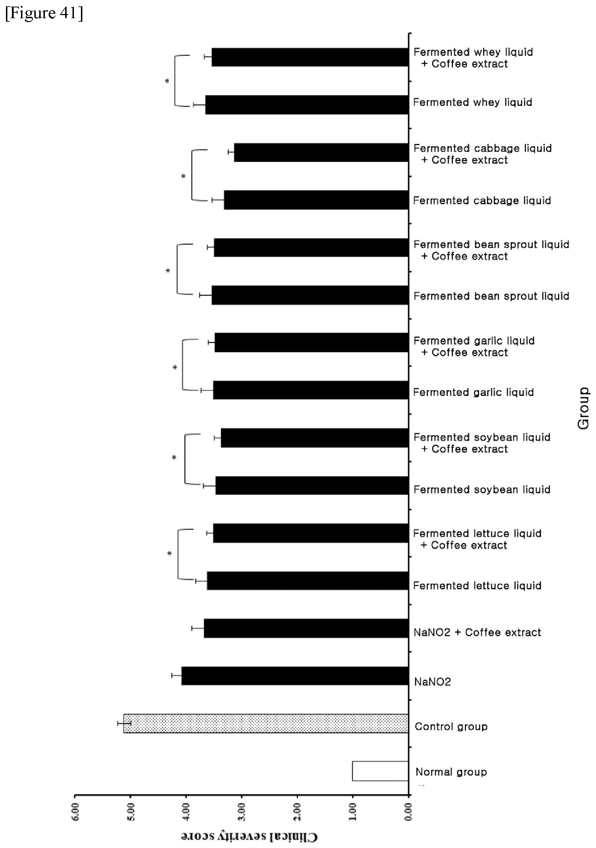

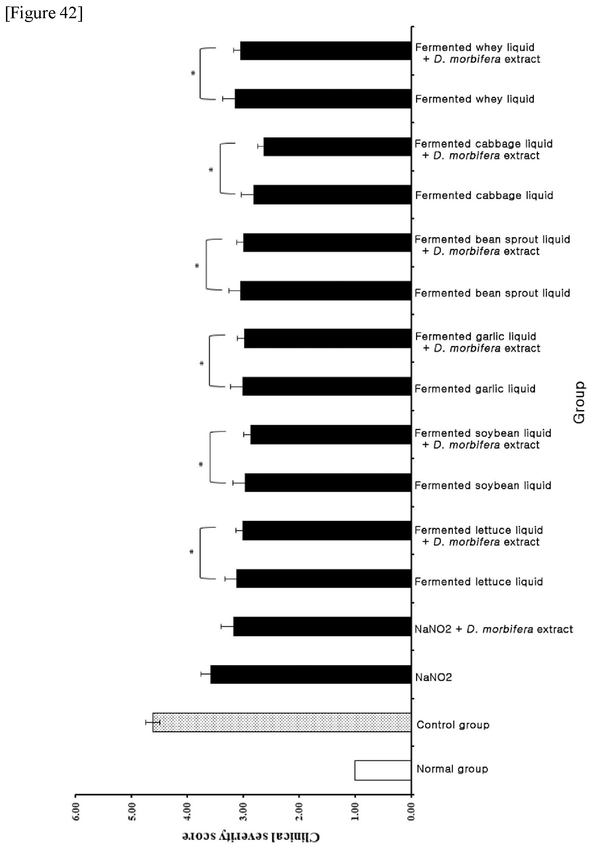

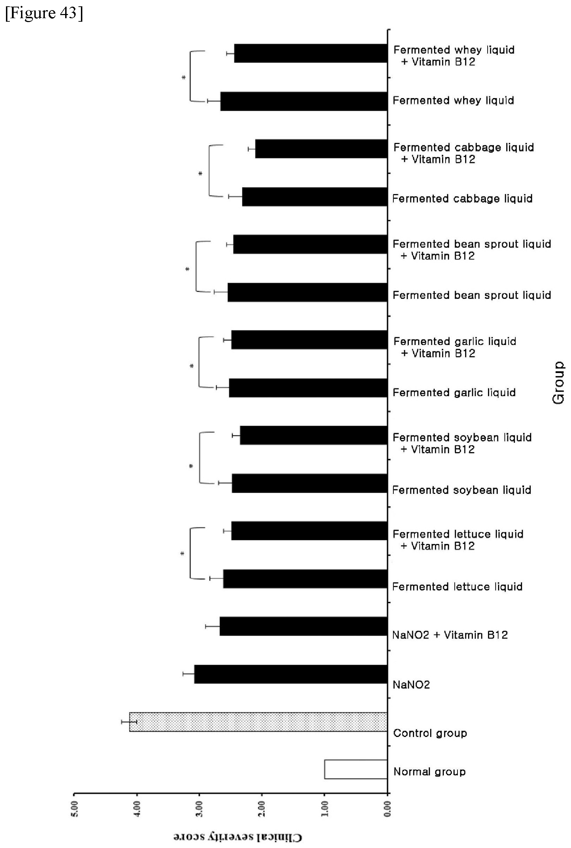

Furthermore, in the MIA-induced osteoarthritis animal model, evaluation of the degree of degenerative arthritis for each group is shown in Figures 38 to 43. In the normal group, which did not receive MIA injection, no abnormal symptoms such as edema were observed. In the MIA-treated control group (MIA), arthritis induction resulted in symptoms accompanied by edema in the left leg. Administration of the test substances led to alleviation of the edema.

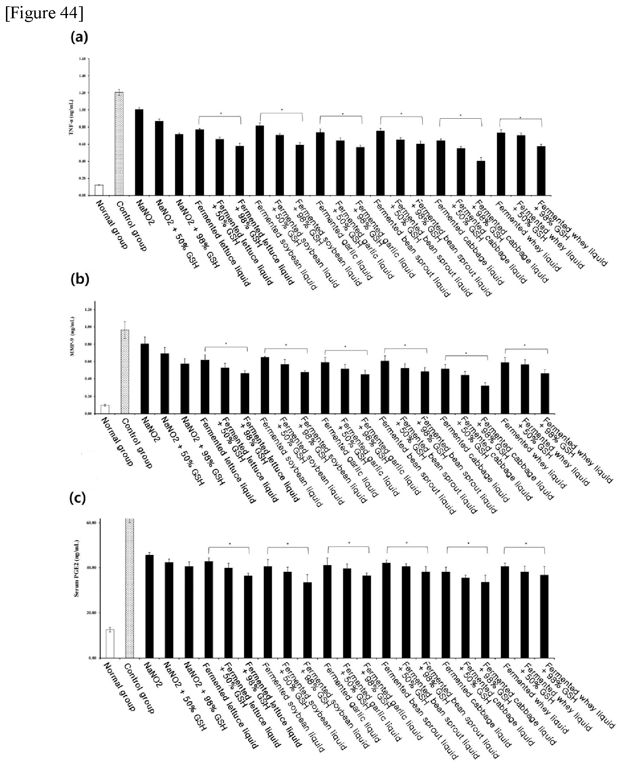

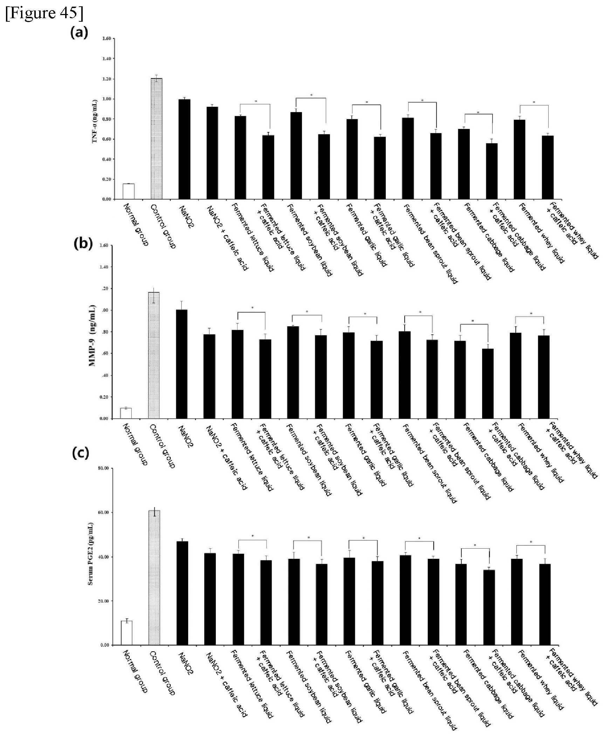

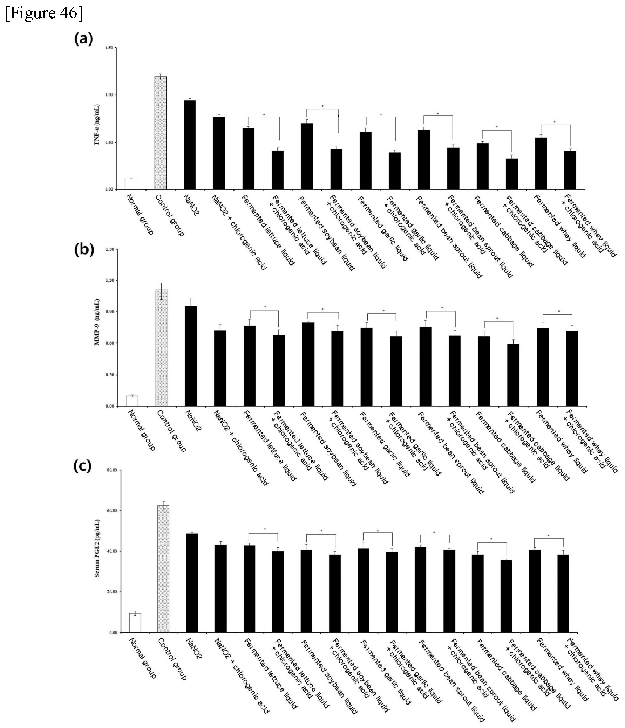

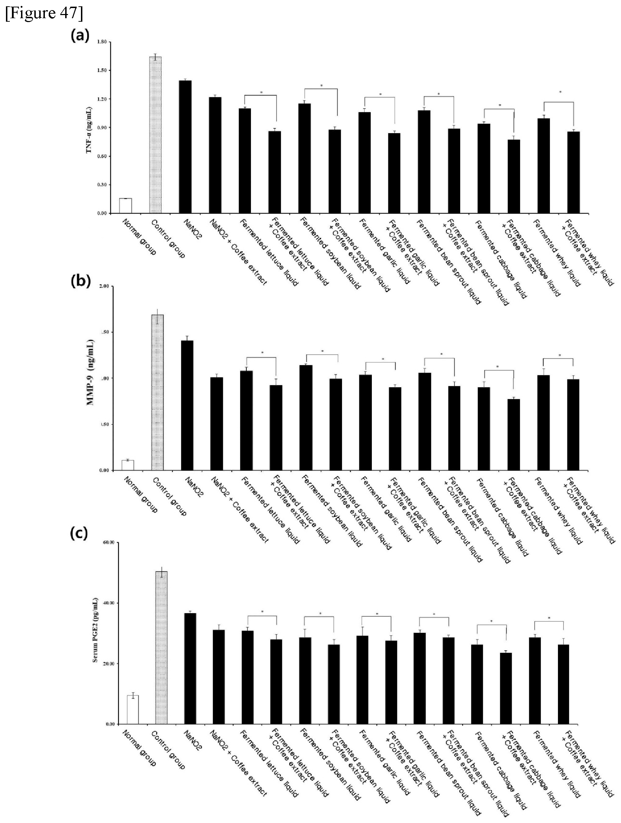

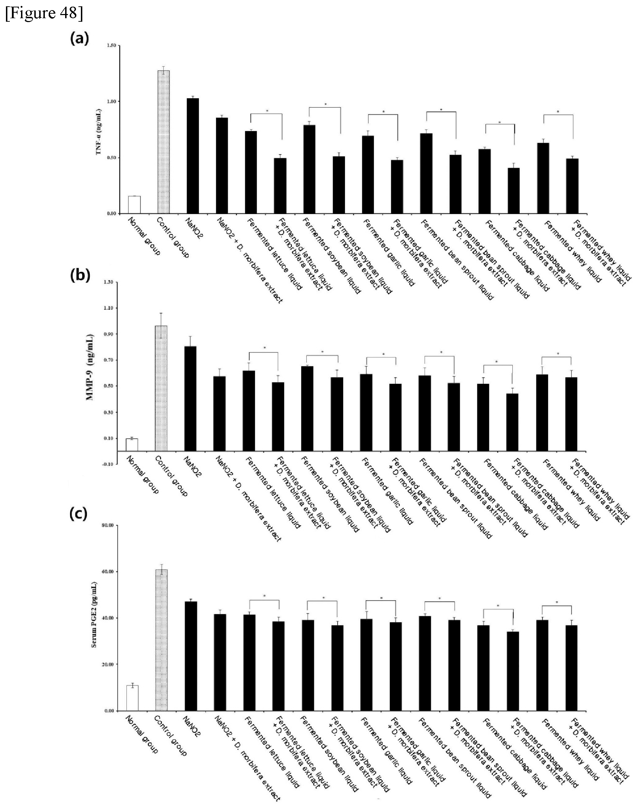

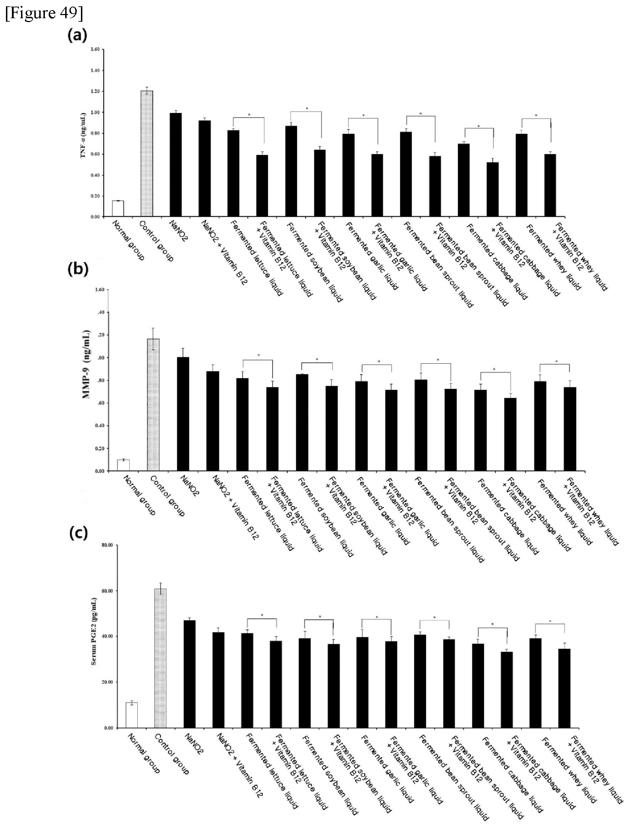

At the end of the experiment, serum was collected and the levels of key inflammatory markers TNF-α, MMP-9, and PGE₂ were measured. All groups administered the test substances showed a reduction compared to the control group, and the groups receiving mixtures of glutathione, caffeic acid, chlorogenic acid, coffee extract, *Dendropanax morbifera* extract, or vitamin B12 in combination with the fermented liquid of natural product exhibited a greater reduction compared to the group receiving only the fermented liquid of natural product (Figures 44 to 49).

### Example 5. Hepatoprotective effect of mixture sample of fermented liquid containing nitric oxide metabolites and glutathione, caffeic acid, chlorogenic acid, coffee extract, Dendropanax morbifera extract, or vitamin B12

In this experiment, seven-week-old male Sprague Dawley rats (SD rats) were purchased from Samtaco and acclimated for one week prior to use. The average body weight of the SD rats was 210.45±1.65 g. The animal housing room was maintained under a 12-hour light/dark cycle, with temperature controlled at 23±2°C and humidity maintained at 50-60%.

### 5-1. Induction of liver injury and group configuration

The hepatotoxicity model using carbon tetrachloride (CCl₄) was established by intraperitoneally administering a mixture of CCl₄ and olive oil in equal volumes at 0.1 mL per 100 g of body weight to induce acute liver damage. The normal group received intraperitoneal injection of olive oil only, without CCl₄. Each experimental group was pretreated by oral administration of 1 mM of the test substance three times on alternate days prior to CCl₄ injection, while the normal group received only distilled water (D.W.) orally. The number of animals per group was determined based on the 3R principle, using the minimum number required to achieve statistical significance.

**[Table 11]**

| Configuration of liver injury experimental groups using mixture of fermented liquid of natural product and glutathione (GSH) | | | | | |
|---|---|---|---|---|---|
| | Group | CCl₄ | Material | Dose | n |
| 1 | Normal group | No | distilled water | - | 5 |
| 2 | Control group | Yes | distilled water | - | 5 |
| 3 | NaNO₂ | Yes | sodium nitrite | 1 mM | 5 |
| 4 | NaNO₂+50% GSH | Yes | sodium nitrite + Yeast extract 50% glutathione | 1 mM | 5 |
| 5 | NaNO₂+98% GSH | Yes | sodium nitrite + 98% glutathione | 1 mM | 5 |
| 6 | Fermented lettuce liquid | Yes | *Lactuca sativa* fermented extract | 1 mM | 5 |
| 7 | Fermented lettuce liquid+50% GSH | Yes | *Lactuca sativa* fermented extract + Yeast extract 50% glutathione | 1 mM | 5 |
| 8 | Fermented lettuce liquid+98% GSH | Yes | *Lactuca sativa* fermented extract + 98% glutathione | 1 mM | 5 |
| 9 | Fermented soybean liquid | Yes | bean fermented extract | 1 mM | 5 |
| 10 | Fermented soybean liquid+50% GSH | Yes | bean fermented extract + Yeast extract 50% glutathione | 1 mM | 5 |
| 11 | Fermented soybean liquid+98% GSH | Yes | bean fermented extract + 98% glutathione | 1 mM | 5 |
| 12 | Fermented garlic liquid | Yes | Garlic fermented extract | 1 mM | 5 |
| 13 | Fermented garlic liquid+50% GSH | Yes | Garlic fermented extract + Yeast extract 50% glutathione | 1 mM | 5 |
| 14 | Fermented garlic liquid+98% GSH | Yes | Garlic fermented extract + 98% glutathione | 1 mM | 5 |
| 15 | Fermented bean sprout liquid | Yes | bean sprout fermented extract | 1 mM | 5 |
| 16 | Fermented bean sprout liquid+50% GSH | Yes | bean sprout fermented extract + Yeast extract 50% glutathione | 1 mM | 5 |
| 17 | Fermented bean sprout liquid+98% GSH | Yes | bean sprout fermented extract + 98% glutathione | 1 mM | 5 |
| 18 | Fermented cabbage liquid | Yes | Cabbage fermented extract | 1 mM | 5 |
| 19 | Fermented cabbage liquid+50% GSH | Yes | Cabbage fermented extract + Yeast extract 50% glutathione | 1 mM | 5 |
| 20 | Fermented cabbage liquid+98% GSH | Yes | Cabbage fermented extract + 98% glutathione | 1 mM | 5 |
| 21 | Fermented whey liquid | Yes | Whey fermented extract | 1 mM | 5 |
| 22 | Fermented whey liquid+50% GSH | Yes | Whey fermented extract + Yeast extract 50% glutathione | 1 mM | 5 |
| 23 | Fermented whey liquid+98% GSH | Yes | Whey fermented extract + 98% glutathione | 1 mM | 5 |

**[Table 12]**

| Configuration of liver injury experimental groups using mixture of fermented liquid of natural product and caffeic acid | | | | | |
|---|---|---|---|---|---|
| | Group | CCl₄ | Material | Dose | n |
| 1 | Normal group | No | distilled water | - | 3 |
| 2 | Control group | Yes | distilled water | - | 3 |
| 3 | NaNO₂ | Yes | sodium nitrite | 1 mM | 3 |
| 4 | NaNO₂+caffeic acid | Yes | sodium nitrite + caffeic acid | 1 mM | 3 |
| 5 | Fermented lettuce liquid | Yes | *Lactuca sativa* fermented extract | 1 mM | 3 |
| 6 | Fermented lettuce liquid+caffeic acid | Yes | *Lactuca sativa* fermented extract+ caffeic acid | 1 mM | 3 |
| 7 | Fermented soybean liquid | Yes | bean fermented extract | 1 mM | 3 |
| 8 | Fermented soybean liquid+caffeic acid | Yes | bean fermented extract + caffeic acid | 1 mM | 3 |
| 9 | Fermented garlic liquid | Yes | Garlic fermented extract | 1 mM | 3 |
| 10 | Fermented garlic liquid+caffeic acid | Yes | Garlic fermented extract + caffeic acid | 1 mM | 3 |
| 11 | Fermented bean sprout liquid | Yes | bean sprout fermented extract | 1 mM | 3 |
| 12 | Fermented bean sprout liquid+caffeic acid | Yes | bean sprout fermented extract + caffeic acid | 1 mM | 3 |
| 13 | Fermented cabbage liquid | Yes | Cabbage fermented extract | 1 mM | 3 |
| 14 | Fermented cabbage liquid+caffeic acid | Yes | Cabbage fermented extract +caffeic acid | 1 mM | 3 |
| 15 | Fermented whey liquid | Yes | Whey fermented extract | 1 mM | 3 |
| 16 | Fermented whey liquid+caffeic acid | Yes | Whey fermented extract + caffeic acid | 1 mM | 3 |

The experimental groups receiving mixtures of chlorogenic acid, vitamin B12, coffee extract, and *Dendropanax morbifera* extract were configured identically to the caffeic acid mixture group shown in the following Table 12.

### 5-2. Serum collection and analysis

Rats used in the experiment were fasted for 12 hours and then anesthetized with isoflurane. Blood was collected from the inferior vena cava and centrifuged at 3,000 rpm for 20 minutes to separate the serum. Serum levels of AST (aspartate transaminase) and ALT (alanine transaminase) were measured using reagents for AST and ALT determination (Asan Pharmaceutical) based on the Reitman-Frankel enzymatic method.

### 5-3. Effect of mixture in acute liver injury model

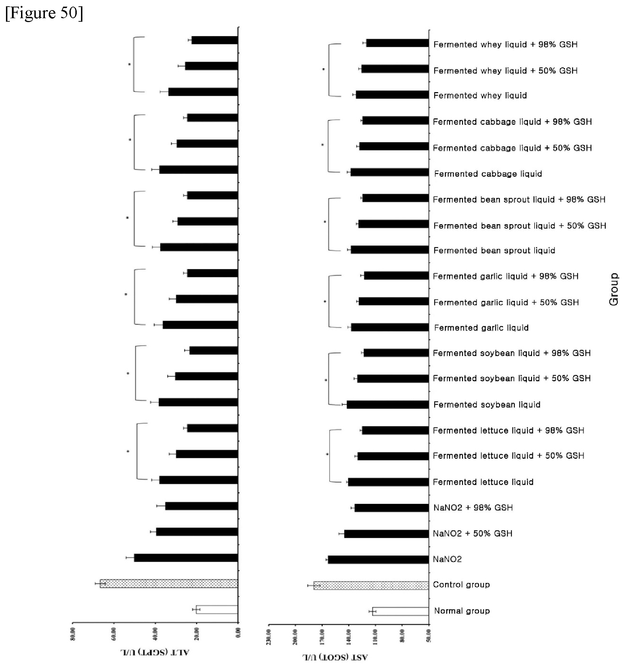

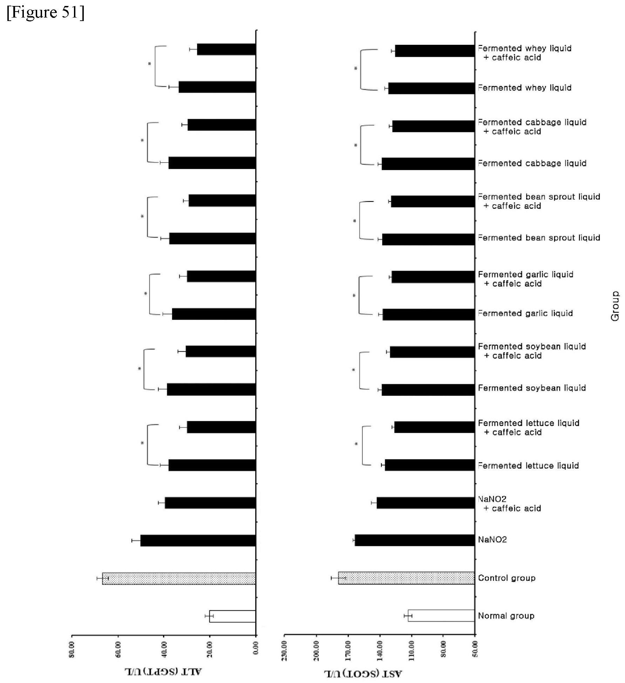

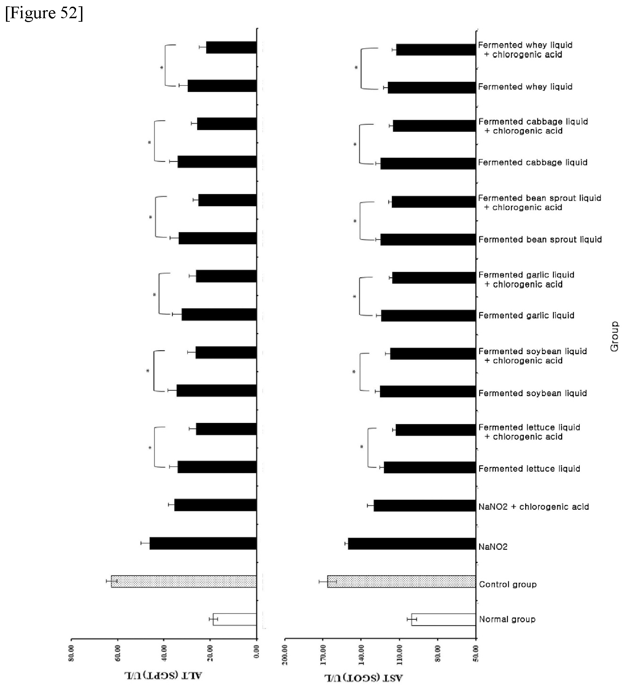

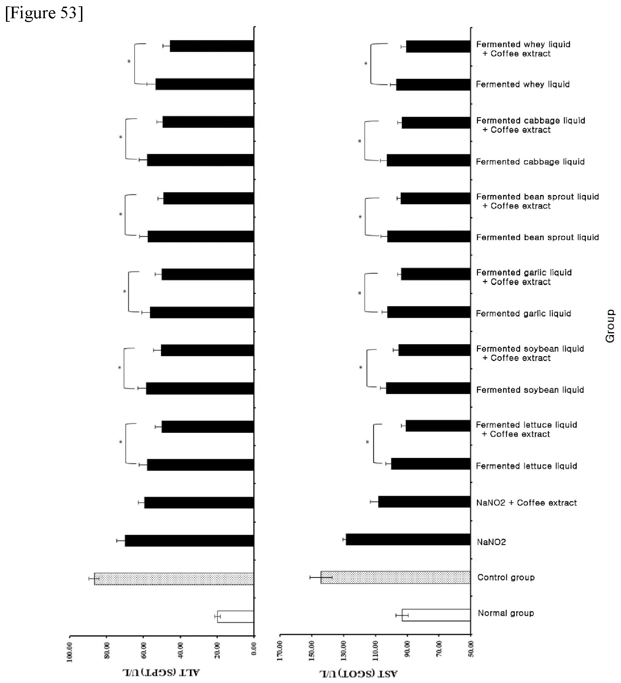

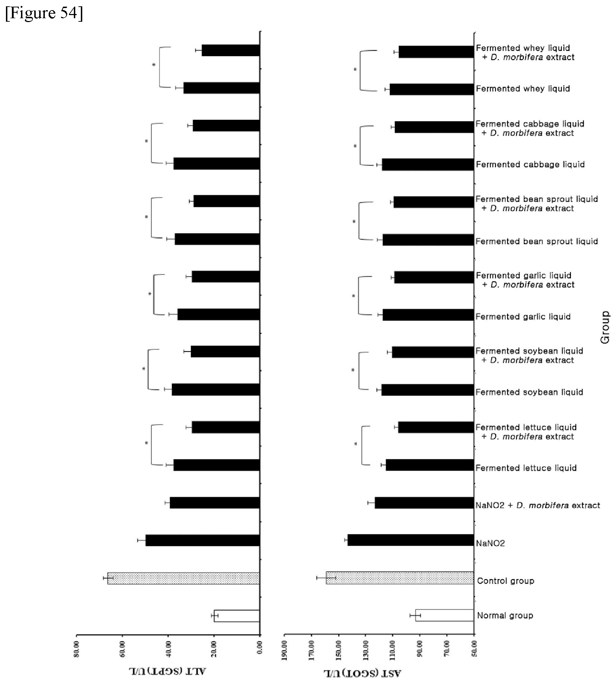

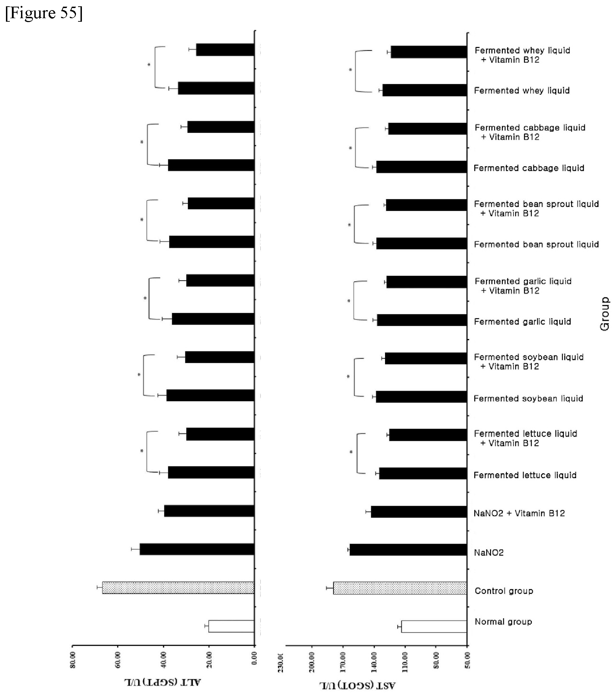

Serum ALT and AST levels are clinically measured as biomarkers of liver health. In the CCl₄-induced hepatotoxicity model, measurement of ALT and AST after the administration of each test substance showed that all treated groups had reduced serum ALT and AST levels compared to the control group. In particular, the groups receiving mixtures of glutathione, caffeic acid, chlorogenic acid, coffee extract, *Dendropanax morbifera* extract, or vitamin B12 in combination with the fermented liquid of natural product showed a greater reduction in serum ALT and AST levels compared to the group receiving only the fermented liquid of natural product (Figures 50 to 55).

### Example 6. Effect of mixture sample of fermented liquid containing nitric oxide metabolites and glutathione, caffeic acid, chlorogenic acid, coffee extract, Dendropanax morbifera extract, or vitamin B12 on amelioration of dermatitis

In this experiment, five-week-old male Balb/c mice were purchased from Samtaco and acclimated for one week prior to use. The average body weight of the Balb/c mice was 20.80±0.82 g, and the test substances were administered orally once daily for 12 days. The animal housing room was maintained under a 12-hour light/dark cycle, with temperature controlled at 23±2°C and humidity maintained at 50-60%.

### 6-1. Preparation of DNFB, induction of atopic dermatitis, and group configuration

After a one-week acclimation period, the dorsal (back) area of Balb/c mice was shaved and the animals were allowed to adapt for 3 days. DNFB (1-fluoro-2,4-dinitrobenzene; Sigma, USA) was prepared by diluting 0.5% DNFB in a solution of acetone and olive oil mixed at a 4:1 ratio. On the fourth day after shaving, 40 µL of 0.5% DNFB was applied to the dorsal area for 4 consecutive days to induce skin sensitization. On the twelfth day of test substance administration after skin sensitization, 20 µL of 0.3% DNFB was applied to the ears (challenge) to induce contact dermatitis.

The experimental groups were configured as shown in the following Tables 13 and 14, and the number of animals per group was determined according to the 3R principle, using the minimum number required to achieve statistical significance. The experimental group receiving a mixture of chlorogenic acid, vitamin B12, coffee extract, and *Dendropanax morbifera* extract was configured identically to the caffeic acid mixture group shown in the following Table 14. The normal group (CON) and the control group received distilled water (D.W.), while the remaining groups were administered their respective test substances orally once daily.

**[Table 13]**

| Configuration of atopic dermatitis experimental groups using mixture of fermented liquid of natural product and glutathione (GSH) | | | | | |
|---|---|---|---|---|---|
| Group | | | Treatment | | n |
| Group | Left | Right | material | Dose | n |
| Normal group | Normal | Normal | distilled water | - | 5 |
| Control group | Normal | 0.3% DNFB | distilled water | - | 5 |
| NaNO₂ | Normal | 0.3% DNFB | sodium nitrite | 1 mM | 5 |
| NaNO₂+50% GSH | Normal | 0.3% DNFB | sodium nitrite + Yeast extract 50% glutathione | 1 mM | 5 |
| NaNO₂+98% GSH | Normal | 0.3% DNFB | sodium nitrite + 98% glutathione | 1 mM | 5 |
| Fermented lettuce liquid | Normal | 0.3% DNFB | *Lactuca sativa* fermented extract | 1 mM | 5 |
| Fermented lettuce liquid+50% GSH | Normal | 0.3% DNFB | *Lactuca sativa* fermented extract + Yeast extract 50% glutathione | 1 mM | 5 |
| Fermented lettuce liquid+98% GSH | Normal | 0.3% DNFB | *Lactuca sativa* fermented extract + 98% glutathione | 1 mM | 5 |
| Fermented soybean liquid | Normal | 0.3% DNFB | bean fermented extract | 1 mM | 5 |
| Fermented soybean liquid+50% GSH | Normal | 0.3% DNFB | bean fermented extract + Yeast extract 50% glutathione | 1 mM | 5 |
| Fermented soybean liquid+98% GSH | Normal | 0.3% DNFB | bean fermented extract + 98% glutathione | 1 mM | 5 |
| Fermented garlic liquid | Normal | 0.3% DNFB | Garlic fermented extract | 1 mM | 5 |
| Fermented garlic liquid+50% GSH | Normal | 0.3% DNFB | Garlic fermented extract + Yeast extract 50% glutathione | 1 mM | 5 |
| Fermented garlic liquid+98% GSH | Normal | 0.3% DNFB | Garlic fermented extract + 98% glutathione | 1 mM | 5 |
| Fermented bean sprout liquid | Normal | 0.3% DNFB | bean sprout fermented extract | 1 mM | 5 |
| Fermented bean sprout liquid+ 50% GSH | Normal | 0.3% DNFB | bean sprout fermented extract + Yeast extract 50% glutathione | 1 mM | 5 |
| Fermented bean sprout liquid+98% GSH | Normal | 0.3% DNFB | bean sprout fermented extract + 98% glutathione | 1 mM | 5 |
| Fermented cabbage liquid | Normal | 0.3% DNFB | Cabbage fermented extract | 1 mM | 5 |
| Fermented cabbage liquid+ 50% GSH | Normal | 0.3% DNFB | Cabbage fermented extract + Yeast extract 50% glutathione | 1 mM | 5 |
| Fermented cabbage liquid+ 98% GSH | Normal | 0.3% DNFB | Cabbage fermented extract + 98% glutathione | 1 mM | 5 |
| Fermented whey liquid | Normal | 0.3% DNFB | Whey fermented extract | 1 mM | 5 |
| Fermented whey liquid+50% GSH | Normal | 0.3% DNFB | Whey fermented extract + Yeast extract 50% glutathione | 1 mM | 5 |
| Fermented whey liquid+98% GSH | Normal | 0.3% DNFB | Whey fermented extract + 98% glutathione | 1 mM | 5 |

**[Table 14]**

| Configuration of atopic dermatitis experimental groups using mixture of fermented liquid of natural product and caffeic acid | | | | | |
|---|---|---|---|---|---|
| Group | | | Treatment | | n |
| Group | Left | Right | material | Dose | n |
| Normal group | Normal | Normal | distilled water | - | 3 |
| Control group | Normal | 0.3% DNFB | distilled water | - | 3 |
| NaNO₂ | Normal | 0.3% DNFB | sodium nitrite | 1 mM | 3 |
| NaNO₂+caffeic acid | Normal | 0.3% DNFB | sodium nitrite + caffeic acid | 1 mM | 3 |
| Fermented lettuce liquid | Normal | 0.3% DNFB | *Lactuca sativa* fermented extract | 1 mM | 3 |
| Fermented lettuce liquid+caffeic acid | Normal | 0.3% DNFB | *Lactuca sativa* fermented extract+ caffeic acid | 1 mM | 3 |
| Fermented soybean liquid | Normal | 0.3% DNFB | bean fermented extract | 1 mM | 3 |
| Fermented soybean liquid+caffeic acid | Normal | 0.3% DNFB | bean fermented extract + caffeic acid | 1 mM | 3 |
| Fermented garlic liquid | Normal | 0.3% DNFB | Garlic fermented extract | 1 mM | 3 |
| Fermented garlic liquid+caffeic acid | Normal | 0.3% DNFB | Garlic fermented extract + caffeic acid | 1 mM | 3 |
| Fermented bean sprout liquid | Normal | 0.3% DNFB | bean sprout fermented extract | 1 mM | 3 |
| Fermented bean sprout liquid+caffeic acid | Normal | 0.3% DNFB | bean sprout fermented extract + caffeic acid | 1 mM | 3 |
| Fermented cabbage liquid | Normal | 0.3% DNFB | Cabbage fermented extract | 1 mM | 3 |
| Fermented cabbage liquid+caffeic acid | Normal | 0.3% DNFB | Cabbage fermented extract +caffeic acid | 1 mM | 3 |
| Fermented whey liquid | Normal | 0.3% DNFB | Whey fermented extract | 1 mM | 3 |
| Fermented whey liquid+caffeic acid | Normal | 0.3% DNFB | Whey fermented extract + caffeic acid | 1 mM | 3 |

### 6-2. Visual assessment

Visual assessment is a clinical evaluation method commonly used for atopic dermatitis, and the severity of atopic dermatitis symptoms was represented by the total sum of scores obtained by evaluating each of the five items. The evaluation items were erythema, pruritus and dry skin, edema and excoriation, erosion, and lichenification. Each item was scored as no symptoms (0), mild (1), moderate (2), or severe (3), and the scores were then summed to give a total ranging from a minimum of 0 to a maximum of 15 points.

### 6-3. Measurement of ear thickness (ear swelling) and spleen index

To assess changes in skin tissue after DNFB application, both ears of the mice were examined. Ear thickness was measured using Vernier calipers (Mitutoyo Co.) at 12, 24, and 48-hour intervals over 3 days.

After measuring ear thickness, the Balb/c mice were sacrificed, and the spleens were excised and weighed. The spleen index was calculated by dividing the spleen weight (mg) by the body weight (g) of each mouse.

### 6-4. Effect of mixture in contact dermatitis model

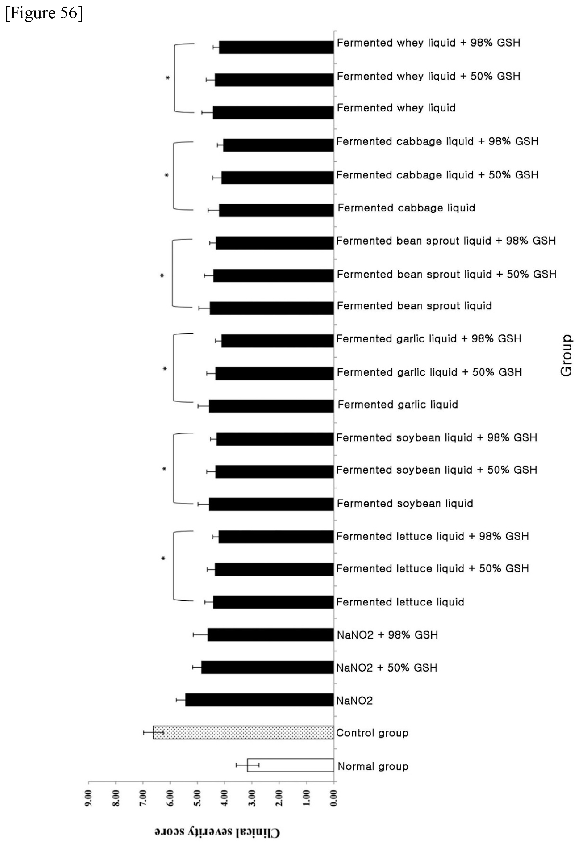

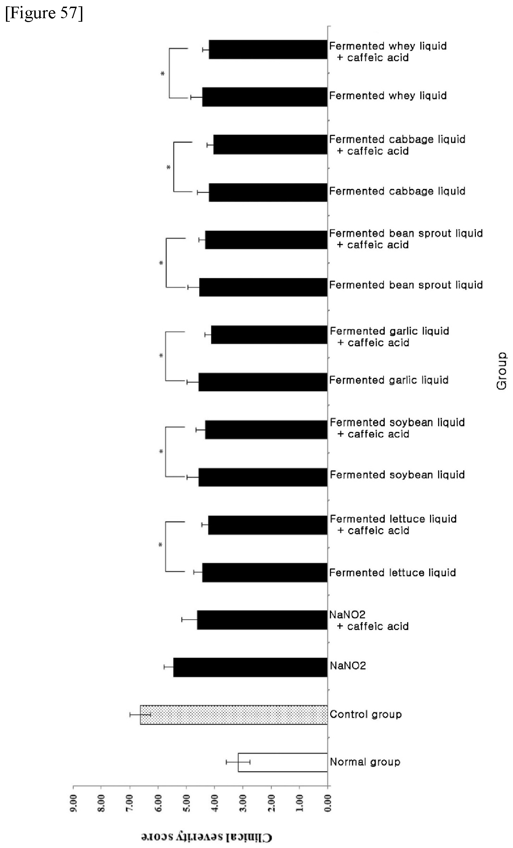

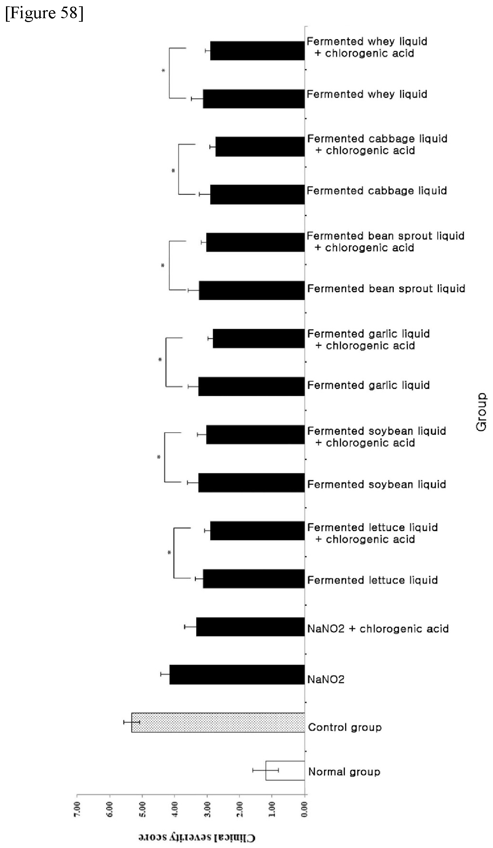

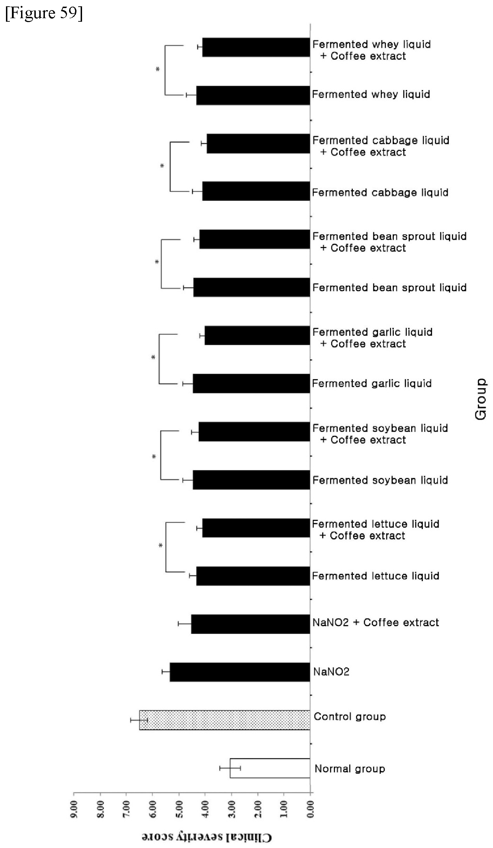

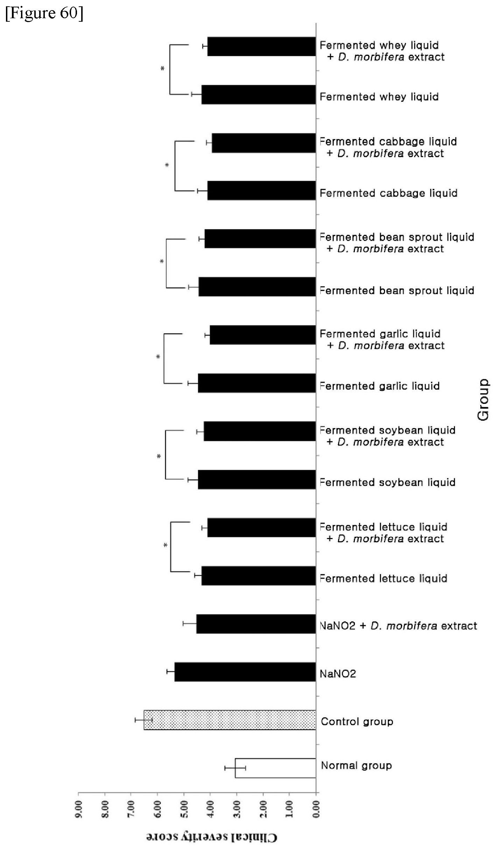

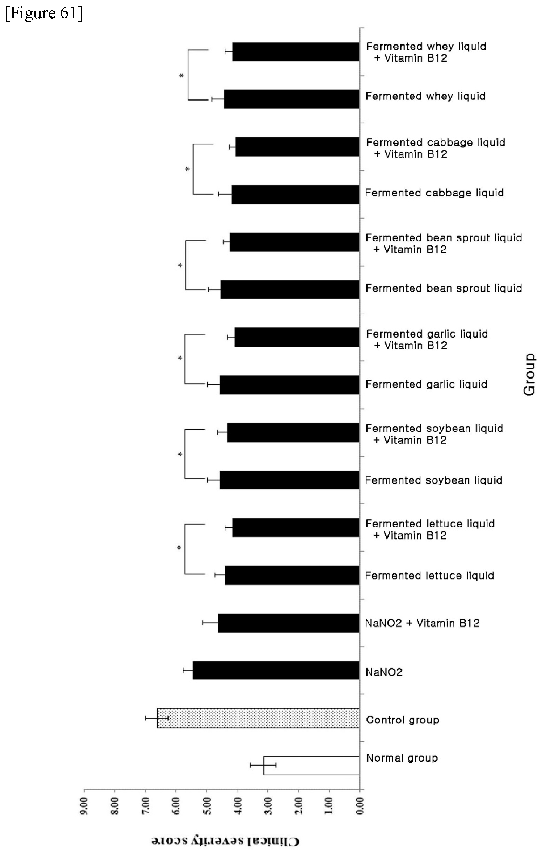

At the end of the experiment, the right ears of each experimental group treated with the test substances were observed to assess morphological changes in atopic dermatitis lesions. In the control group treated with DNFB only, atopic skin changes such as erythema, dry skin, erosion, edema, and excoriation were observed compared to the untreated left ear (Figures 56 to 61). In the test substance-treated groups, these atopic skin changes such as erythema, dry skin, erosion, edema, and excoriation compared to the left ear that received no treatment were milder than in the control group, and it was also found that the groups receiving mixtures of glutathione, caffeic acid, chlorogenic acid, coffee extract, *Dendropanax morbifera* extract, or vitamin B12 in combination with the fermented liquid of natural product showed less pronounced atopic skin changes compared to the group receiving only the fermented liquid of natural product.

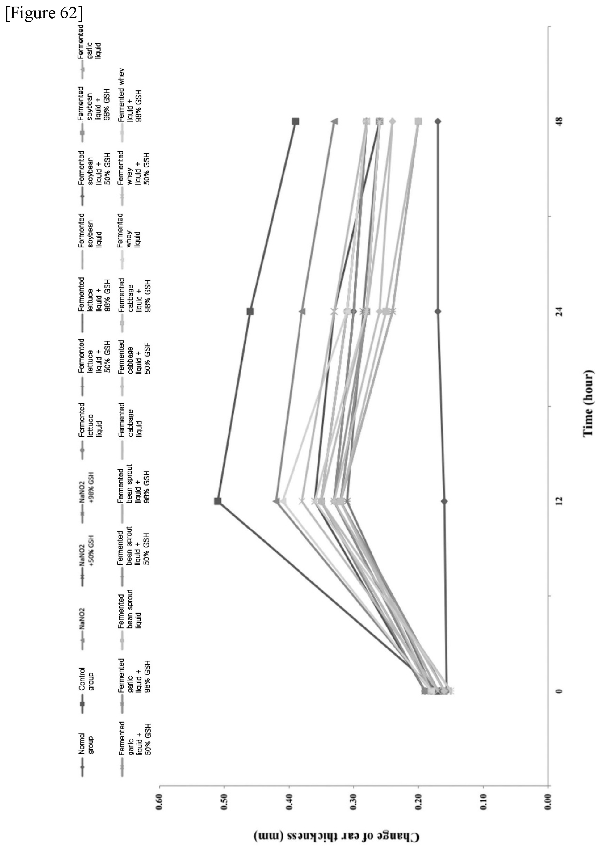

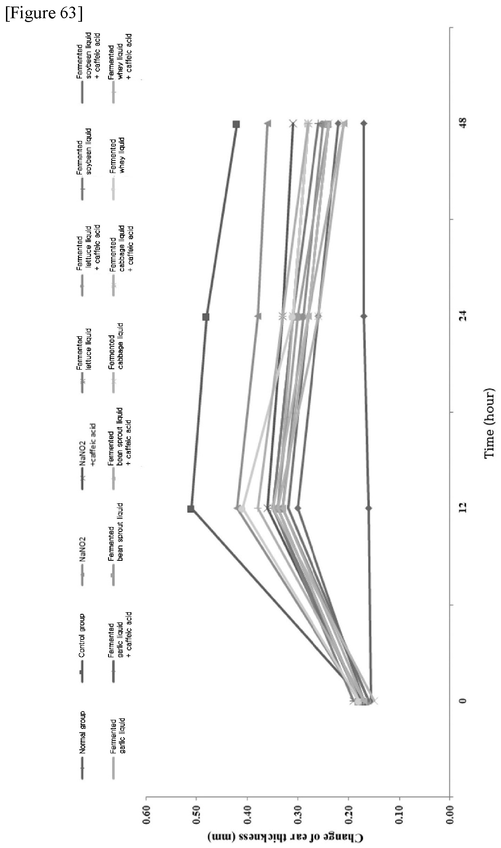

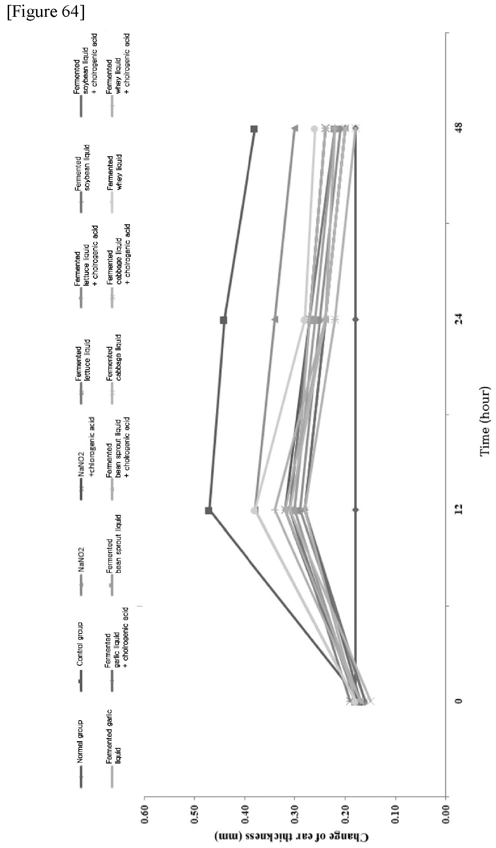

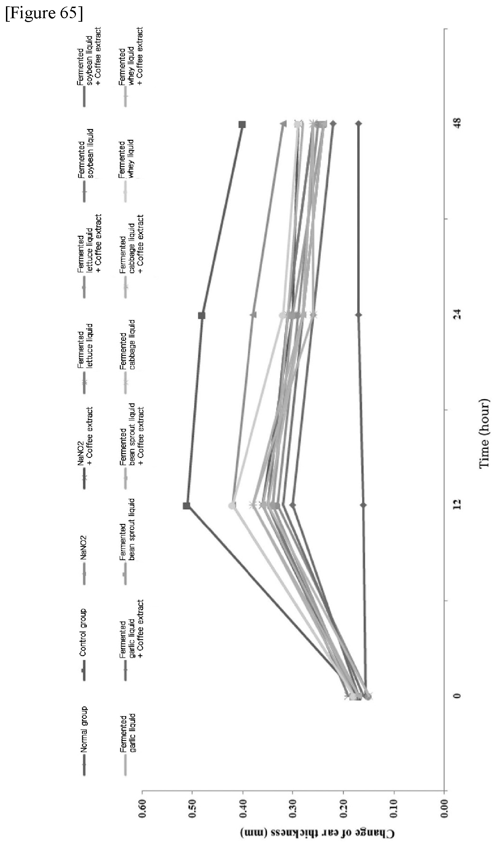

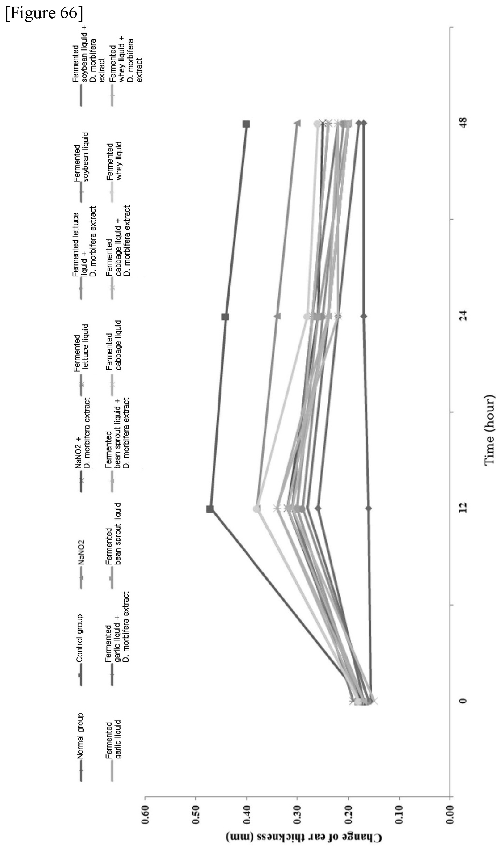

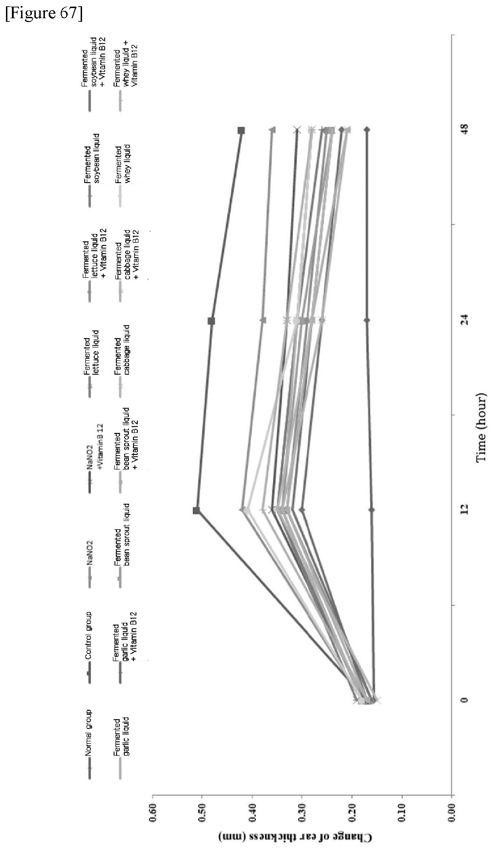

Furthermore, at the end of the experiment, observation of the right ears treated with each test substance showed that ear thickness was reduced in all test substance groups compared to the control group. In particular, the groups receiving mixtures of glutathione, caffeic acid, chlorogenic acid, coffee extract, *Dendropanax morbifera* extract, or vitamin B12 in combination with the fermented liquid of natural product showed a greater reduction in ear thickness than the group receiving only the fermented liquid of natural product (Figures 62 to 67).

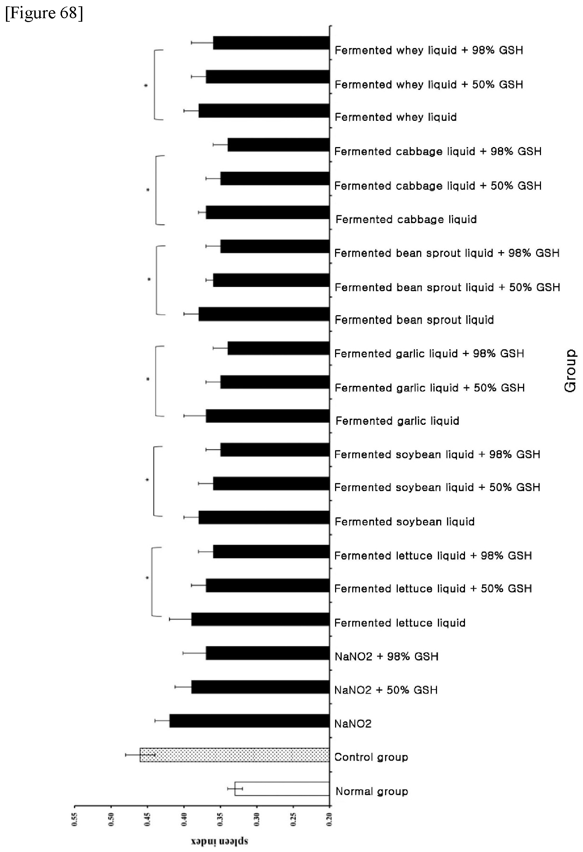

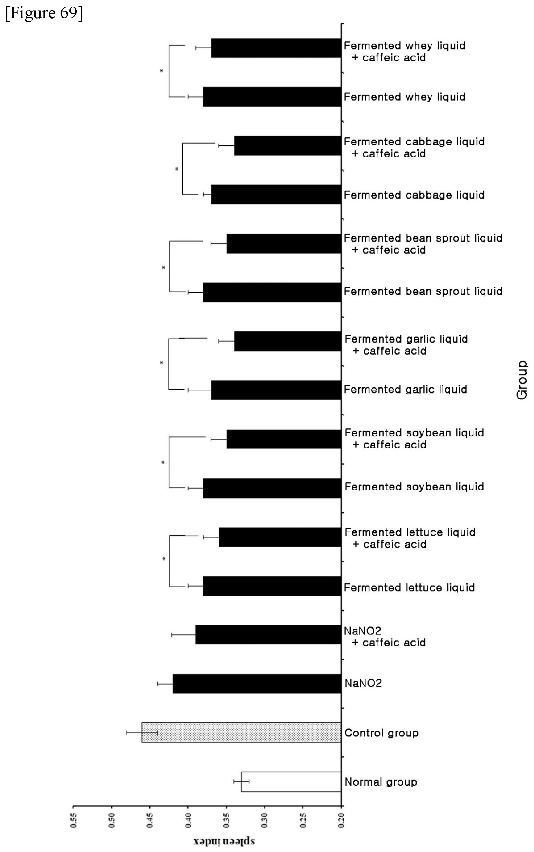

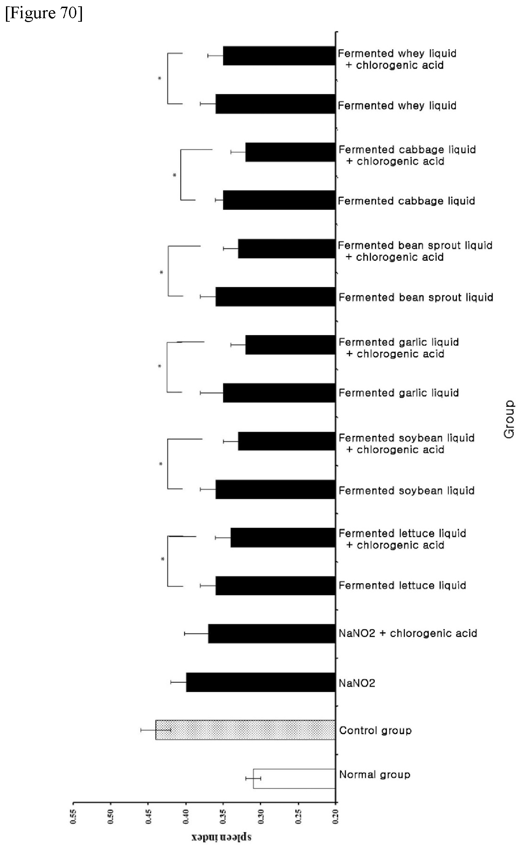

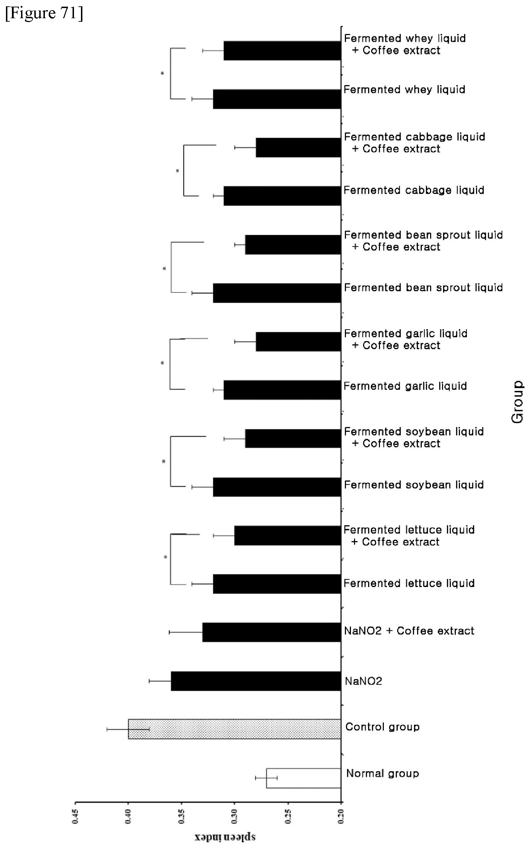

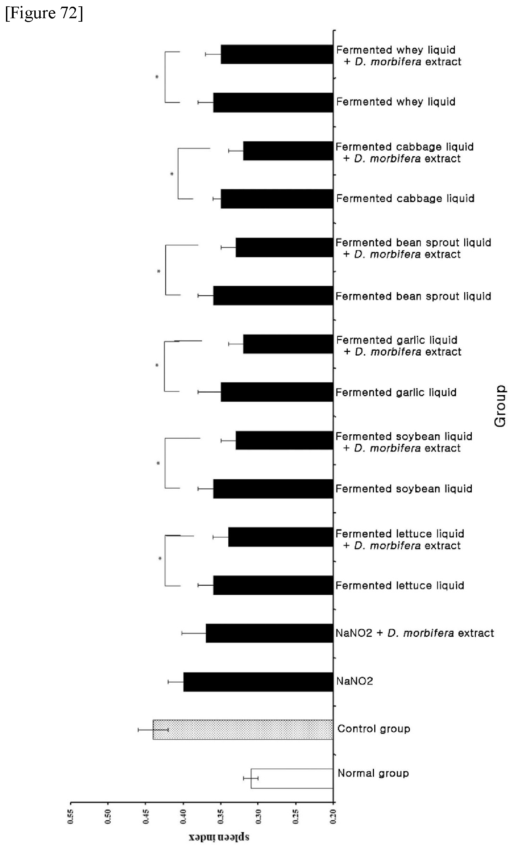

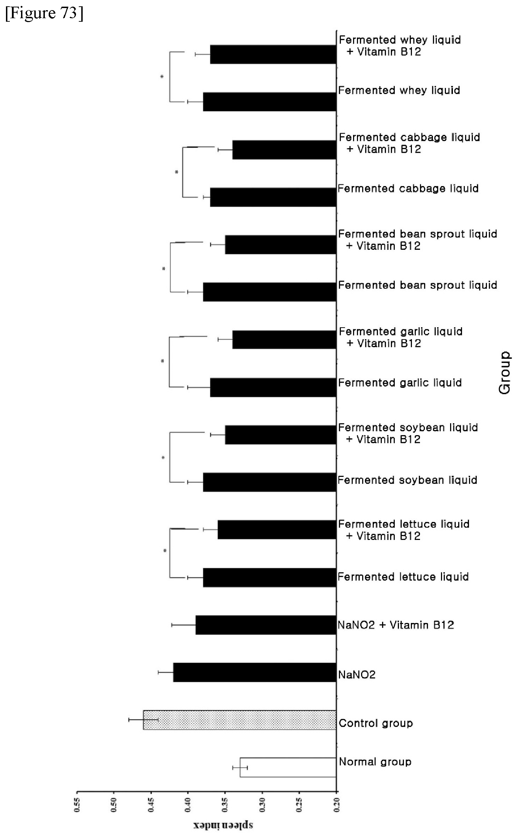

Furthermore, at the end of the experiment, measurement of spleen weight relative to body weight in each test group showed that the spleen index was reduced in all test substance groups compared to the control group. In particular, the groups receiving mixtures of glutathione, caffeic acid, chlorogenic acid, coffee extract, *Dendropanax morbifera* extract, or vitamin B12 in combination with the fermented liquid of natural product showed a greater reduction in spleen index than the group receiving only the fermented liquid of natural product (Figures 68 to 73).

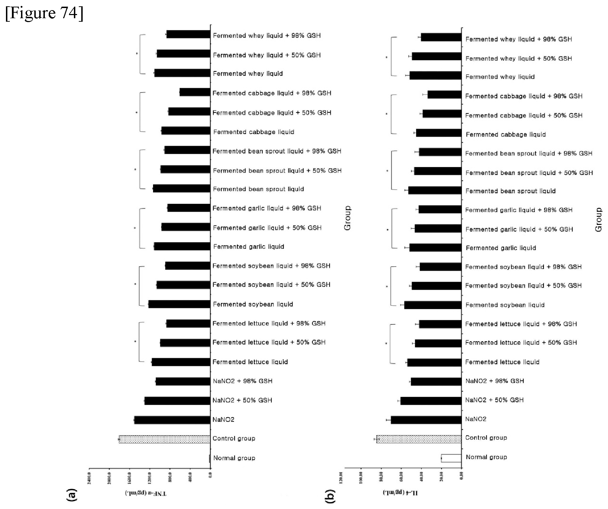

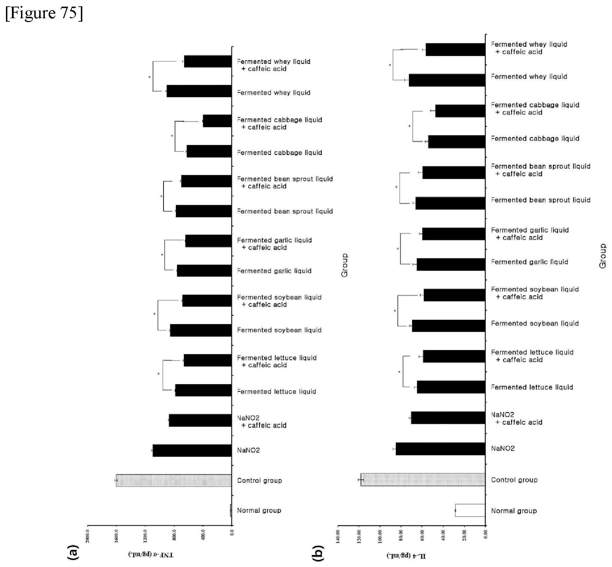

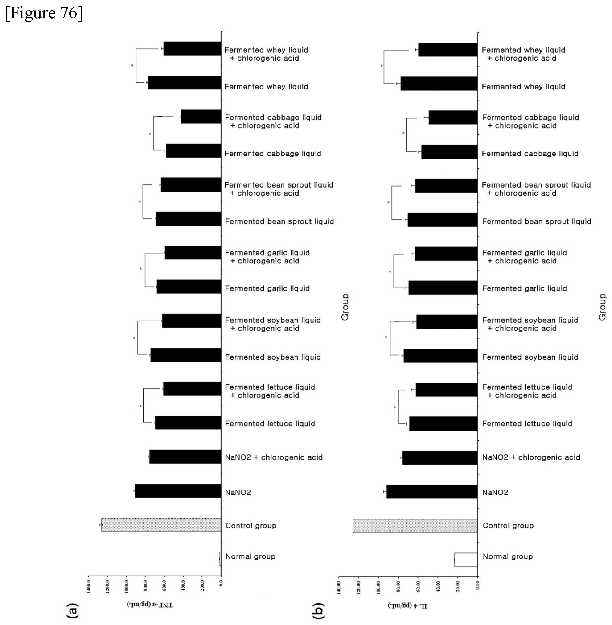

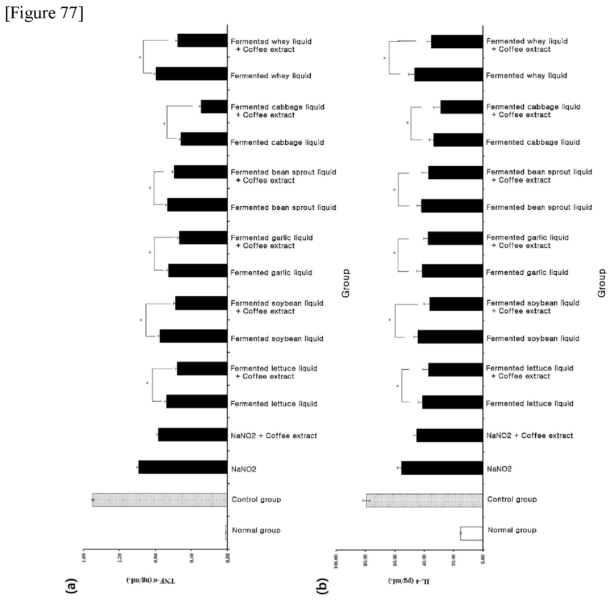

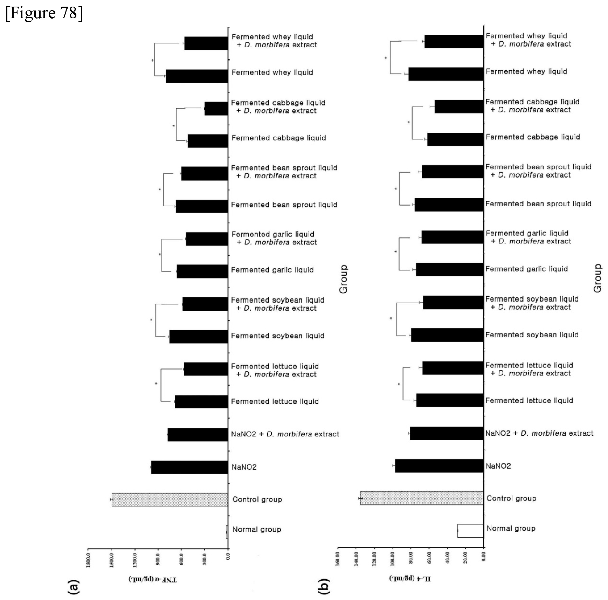

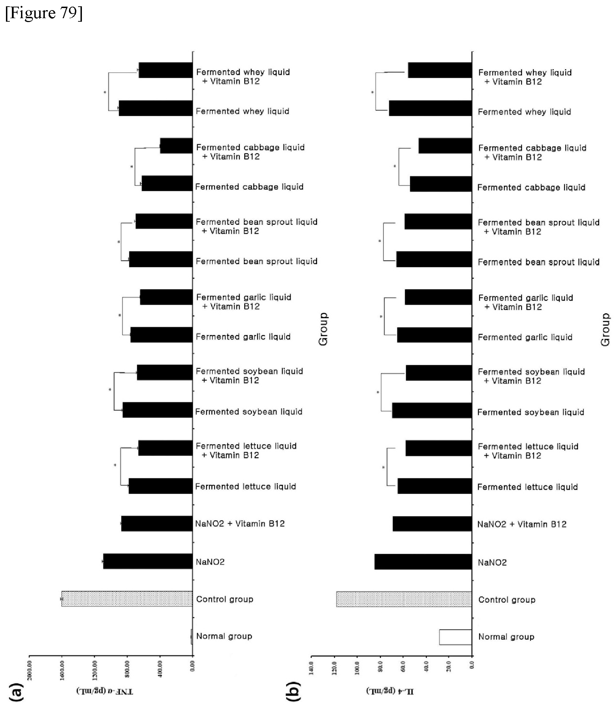

Furthermore, measurement of serum TNF-α and IL-4 levels showed that both TNF-α and IL-4 levels were reduced in all test substance groups compared to the control group. In particular, the groups receiving mixtures of glutathione, caffeic acid, chlorogenic acid, coffee extract, *Dendropanax morbifera* extract, or vitamin B12 in combination with the fermented liquid of natural product showed lower levels of TNF-α and IL-4 than the group receiving only the fermented liquid of natural product (Figures 74 to 79).

## Claims

1. A pharmaceutical composition for preventing or treating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.

2. The pharmaceutical composition according to claim 1, wherein the active ingredients inhibit formation of carcinogenic N-nitrosamines in stomach and promote generation of nitric oxide in intestines.

3. The pharmaceutical composition according to claim 1, wherein the fermented natural product in which nitric oxide metabolites are fixed and stabilized is prepared by a method comprising adding a fermentation strain to a nitrogen-containing natural product and carrying out fermentation at a temperature of 18 to 35°C and a dissolved oxygen concentration of 0.03 to 0.1 mg/L for 2 to 30 days.

4. The pharmaceutical composition according to claim 3, wherein the nitrogen-containing natural product is soybean, garlic, bean sprout, cabbage, or whey.

5. The pharmaceutical composition according to claim 1, wherein the composition further comprises a pharmaceutically acceptable carrier, excipient, or diluent in addition to the active ingredients.

6. A functional health food composition for preventing or ameliorating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.

7. The functional health food composition according to claim 6, wherein the composition is prepared in any one formulation selected from powder, granule, pill, tablet, capsule, candy, syrup, and beverage

8. A veterinary composition for preventing or treating hypertension, enteritis, stress-induced irritable bowel syndrome, arthritis, acute liver injury, or dermatitis comprising, as active ingredients, a fermented natural product in which nitric oxide metabolites are fixed and stabilized, and any one selected from the group consisting of glutathione, caffeic acid, chlorogenic acid, vitamin B12, coffee extract, and *Dendropanax morbifera* extract.
